# EUROPEAN PATENT APPLICATION

(11) **EP 4 191 596 A1**
(43) Date of publication of application: **07.06.2023**
(21) Application number: 22211835.8
(22) Date of filing: 06.12.2022
(51) Int. Cl.: G16H 10/60, G16H 20/30

(54) **SYSTEM OF GENERATING A USER'S REHABILITATION PLAN AND A WAY OF ASSIGNING PROFILE PARAMETERS OF SENSORY PROCESSING DISORDERS**

(30) Priority: 06.12.2021 PL 43975621
(71) Applicant: Empis & Sensum Mobile Sp. z o.. Sp.k., 02-758 Warszawa (PL)
(72) Inventor: DANILUK-PRZYROWSKA, Damiana, 03-425 Warszawa (PL); PRZYROWSKI, Zbigniew, 03-425 Warszawa (PL); PRZYROWSKI, Mateusz, 03-672 Warszawa (PL)
(74) Representative: Marcinska-Porzuc, Aleksandra

(57) **Abstract**

This invention relates to a method for assigning a user to a diagnostic profile assigned to a predefined measurement scale of sensory processing disorders, a method for assigning profile parameters of sensory processing disorders corresponding to at least one value on a measurement (diagnostic) scale sensory processing disorders, and a method for generating and presenting the diagnostic profile to the user.

## Description

### Field

This invention relates to a method for assigning a user to a diagnostic profile assigned to a defined measurement scale of sensory processing disorders, a method for assigning profile parameters of sensory processing disorders corresponding to at least one value on a measurement (diagnostic) scale of sensory processing disorders, and a method for generating and presenting a diagnostic profile to a user.

Sensory integration is a process by means of which the brain learns to recognize, segregate, and interpret sensory stimuli based on earlier experiences. Processes of sensory integration start already during the fetal period and last for the whole life. If specific skills do not develop in specific stages, difficulties in a child's functioning may arise in the following development stages.

Sensory integration allows for an appropriate organization of stimuli arriving at the brain from sensory receptors. Such signals include, for example, vision, hearing, touch, balance, or feeling of movement. To interpret them correctly, the brain has to recognize and classify them. If the process fails at any stage, then therapy is necessary.

Disorders of sensory integration will not pass automatically, this meaning that they have to be removed by appropriate rehabilitation exercises. The sensory integration therapy is based on working by playing with various equipment. During a session, a child performs various activities that are entertaining and interesting and simultaneously stimulate the nervous system with stimuli that the brain classifies and integrates.

Sensory integration therapy is intended to affect the functions of the sensory systems and processes occurring in the nervous system. The activities taken during play are intended to improve the child's functioning in such areas as kinesthetic capability, visual-motor coordination, development of speech, development of concentration ability, the appropriate level of excitation, appropriate falling asleep, learning, etc.

Relying on data about the number of children with disorders accompanying the disorders of sensory integration, one may estimate that 2% of the population faces this problem. Based on the data from the Centers for Disease Control and Prevention, in the U.S., one out of 88 children suffers from autism, and 80% of them are additionally diagnosed with accompanying disorders of sensory integration. In Poland, autism is diagnosed in one child per c.a. 300, and this means autism in c.a. 20 thousand children, out of which c.a. 80% have disorders of sensory integration. According to international statistics, 3-7% of all children have ADHD (although in the U.S., ADHD is diagnosed in c.a. 10% of the population), and c.a. 60% of them have disorders of sensory integration (Miller L.J. 2006, Sensational Kids).

U.S. studies (Miller, Koomar, Benson, Schoen, Brett-Green, Reale, 2006 ) show that 27 % of examined children with sensory processing disorders (so-called SPD) have a so-called generalized SPD that is a combination of all disorder subtypes. This fact makes diagnosing such children a complex process, and the therapy should be intensive.

Statistically, in the average population, the problem relates to 10-15% of children with disorders of sensory integration processes. Observations of the authors of the present invention allow risking a statement that the sickness rate is increasing. Probably, at least partially, it results from widening the diagnostic criteria that make it possible to include cases that had been classified otherwise previously and a more apt diagnostics of these disorders.

The key need of patients suffering from sensory processing disorders (also SPD or SI hereinafter) is a fast apt diagnosis and an appropriately selected therapy conducted with the use of adequate tools. The therapy should satisfy the patient's needs and, thereby, efficiently normalize the processes of sensory integration. For patients who do not have access to specialized care, the key need is access to appropriate therapies. Sensory integration (SI) therapists and pediatricians expect normalized diagnostic criteria that make it possible to use uniform diagnostic rules for SI disorders and tools, making it possible to diagnose the symptoms early and thoroughly. The needs of legal guardians are similar to those of therapists, but the scale is somewhat different due to the fact that they mainly expect an exercise program and a set of tools to be used at home. The caretakers need access to information and support in the form of a plan for supporting the family.

It is estimated that in 2015 in Poland, c.a. 400 000 children were born, 15 000 were in an at-risk group of SI disorders, and 1 200 of them should be SI diagnosed for sure. Most of them will be diagnosed at the age of c.a. 6, so late enough to lose a chance for a fast therapeutic intervention overtaking the cumulation of problems reducing the chances for efficient education and work in the future. According to Statistics Poland's population prognosis up to 2026, the average birth number is estimated to be c.a. 300 000, thus giving in total in the next 5 years 51 000 children with a risk of SI disorders and 40 800 children with diagnosed problems.

For this group of patients, early diagnosis of deviations from the typical development is beneficial because it makes it possible to exploit the child's potential maximally to apply focused interventions, and it contributes to decreasing the prevalence of accompanying diseases.

The average waiting time for a therapy refundable from public funds in a specialized therapeutic room in Poland is from one to two years, and the waiting time for a diagnostic consultation in private units is 1 to 4 months. The waiting time for a course for SI therapists is from 2 to 3 years. In many regions, there is a lack of units of this type, while existing facilities, mainly located in big urban agglomerations, are not able to meet the relevant demands. In periphery regions, parents and children are left alone facing the problem, particularly families of smaller incomes who cannot afford commuting and therapy in remote centers. Moreover, a noticeable problem is the duration of the therapy that is conducted once a week or less in Poland, whereas experiences of American therapists with SPD suggest that an intensive therapy of 30 sessions, considered as a real minimum (60 minutes 3-5 times a week), is more beneficial for the child's progress. Also, the involvement of parents in the therapy is not sufficient and usually limited to occasional 'corridor' contacts, the result of which is the lack of sufficient knowledge about the actual child's progress and the information about the real-life factors that affect the therapy process. SI therapy is focused on the family, not only on the child. Therefore, cooperation with parents is necessary. The disorders of sensory integration are a significant problem affecting all daily-life aspects.

The total cost of treating neuronal-based disorders in Europe was EUR 798 billion, out of which 37% covered the costs of direct medical care and 23% costs of indirect medical care.

Therefore, the aim of the present invention is to propose a remote computer-assisted diagnostics of small children for overall disorders of sensory processes without the necessity of visiting a physician or a therapist by computer-based classifying sensory disorders, assigning them to a relevant value on the measurement scale available in databases, and proposing an appropriate diagnostic profile for a given disorder in the form of an assigned a rehabilitation plan including a schedule with a set of rehabilitation exercises that are sent to a computer-based device of a caregiver to be displayed on the screen of this device. Thus, the aim of the invention is to propose unlimited access to rehabilitation exercises that will largely eliminate deficiencies in undertaking rehabilitation for SI disorders.

The solution of the invention is dedicated to children with a risk of sensory processes disorders without any medical diagnoses as well as for children with already diagnosed development disorders such as autism, ADHD, or genetic disorders such as fragile X syndrome, Down syndrome, and to children with mental impairment, risk of dyslexia, or already diagnosed dyslexia.

This aim has been reached by a system for generating a user's rehabilitation plan, including assigning a user to a diagnostic profile assigned to a defined measurement scale of sensory processing disorders and a method for assigning profile parameters of sensory processing disorders corresponding to at least one value on a measurement (diagnostic) scale of sensory processing disorders.

The invention relates to a system for generating a user's rehabilitation plan, including assigning a user to a diagnostic profile assigned to a defined measurement scale of sensory processing disorders, communicating to a computer network with the use of computer-based devices, and accessing data via access means, including:
c) Obtaining unit data covering a user's data retrieved from:
   i) a Test of Sensory Processing Disorders,
   ii) a questionnaire,
d) Processing the unit data in order to generate at least one set of computerized profile parameters of the degree of sensory processing disorder,
e) Obtaining reference/control data that includes at least one set of predefined parameters corresponding to at least one defined measurement scale,
f) Comparing at least one set of computerized profile parameters with at least one set of predefined parameters corresponding to at least one measurement scale to establish a degree of the user's sensory processing disorder,
g) Assigning the diagnostic profile corresponding to at least one value on the measurement scale to at least one set of computerized user profile parameters,
h) Generating a rehabilitation plan for the user assigned to the given diagnostic profile using a computer-based device.

According to the invention, the unit data includes data related to the degree of the sensory processing disorder, such as:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii) balance (B),
viii) praxes (P),
ix) reflex integration (RI),
x) visual-motor coordination (VMC),
xi) distribution of antigravity muscles tension (DAMT).

The common access means sends, to a computer-based device associated with a user, an electronic invitation to perform a user electronic questionnaire/reconnaissance via a network, wherein every question of the user electronic questionnaire/reconnaissance is configured to measure behavioral features of the user and to assign them to a defined measurement scale of sensory processing disorders.

According to the invention, the system may comprise additional steps of:
a) Obtaining variables describing behavioral features of the user retrieved from the reconnaissance,
b) Processing the variables describing behavioral features in order to qualify the user for further diagnostic tests and/or a consultation with a sensory integration therapist.

Also, the system may include an additional step of:
i) Generating a user profile.

In the system of the invention, the reference/control data that consists of at least one set of predefined parameters assigned to a given value of the measurement scale is predefined and delivered by the common access means according to a method as defined in the claims. The user profile is created and stored on a server, wherein the server is a part of the common access means. Preferably, a user ID identifier is assigned to the user profile. The user profile is created using predefined elements of user profiles. Preferably, the user profile includes at least one element from the following: personal data, a degree of sensory processing disorder, a diagnosis, a set of computerized user profile parameters, a set of predefined parameters generated for the given user, a value of the measurement scale to which the given user is assigned, a diagnostic profile and a rehabilitation plan assigned to these parameters.

A rehabilitation plan is assigned to every diagnostic profile for a given disorder, comprising a schedule and/or at least one rehabilitation exercise. Moreover, the diagnostic profile corresponds to the user age category or depends on the user age category. The diagnostic profile is generated for a defined time period, preferably for 4 weeks. The generated diagnostic profile includes rehabilitation exercises for at least one of the following sensory processing disorders:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii)balance (B),
viii) praxes (P),
ix) reflex integration (RI),
x) visual-motor coordination (VMC),
xi) distribution of antigravity muscles tension (DAMT),

The at least one assigned rehabilitation exercise or the set of rehabilitation exercises is presented to the user in the form of graphical representations using a computer-based device associated with the user.

The diagnostic profile, including the effects of performing the at least one rehabilitation exercise or the set of rehabilitation exercises, along with the assigned thereto rehabilitation plan, is reported periodically to the common access means in the form of a computer file containing the user ID identifier.

After a predefined time period, a control test is generated verifying the effects of performing the at least one rehabilitation exercise or the set of rehabilitation exercises of the given diagnostic profile assigned to the given user.

Moreover, the system comprises a step of analyzing the reported diagnostic profiles, including the effects of performing the at least one rehabilitation exercise or the set of rehabilitation exercises along with the assigned thereto rehabilitation plan and/or results of users' control tests, wherein the rehabilitation progress of the users belonging to the same age category are grouped into several groups - groups assigned to the same or a similar value on the measurement scale and/or to a given diagnostic profile.

Moreover, the system comprises a step of updating the diagnostic profile based on assigning:
- the user to another value on the measurement scale, and/or
- the user to another diagnostic profile, and/or
- the rehabilitation exercise and/or exercises to a new or another value on the measurement scale of sensory processing disorders, and/or
- adding at least one new rehabilitation exercise to the given diagnostic profile.

The step of updating the diagnostic profile is executed in databases of the common access means and is transferred to the user's computer-based device by the common access means in the form of a computer file containing the user ID identifier.

Preferably, the system comprises, moreover, a step of generating a new diagnostic profile and a new, assigned thereto, at least one rehabilitation exercise or a set of rehabilitation exercises, and/or extending the execution time for this rehabilitation plan.

The correctness of the execution of the rehabilitation exercise is assessed according to a predefined point scale.

The invention also relates to a method for assigning profile parameters of sensory processing disorders corresponding to at least one value on a measurement (diagnostic) scale of sensory processing disorders, accessing data resources in the common access means storing sets of predefined profile parameters of many users, and comprising steps of:
a) receiving a user's unit data and assigning a first weight thereto,
b) receiving data about the number of users and assigning a second weight thereto,
c) receiving data about the age of the users and assigning a third weight thereto,
d) receiving data about the duration of the user's therapy and assigning a fourth weight thereto,
e) calculating reference/control data being at least one set of predefined profile parameters for the given user corresponding to at least one value on a defined measurement scale based on the user unit data, the number of users, the age of users, the therapy duration, and the assigned weights,
f) generating reference/control data for the given user.

Moreover, the method comprises a step g) of receiving data about at least one rehabilitation exercise or set of rehabilitation exercises established for a given diagnostic profile corresponding to a selected value on a defined measurement scale and assigning a fifth weight thereto.

Preferably, the user's unit data is data retrieved from the Test of Sensory Processing Disorders and/or the user's personal questionnaire. At least one set of computerized profile parameters of the degree of the user's sensory processing disorders is generated from the user's unit data.

If the computerized profile parameters of the degree of the sensory processing disorder of a given user can't be assigned to any value on the measurement scale, a new value is created on the measurement scale, and to this new value, computerized profile parameters of the degree of the user's sensory processing disorders are assigned that are then preliminarily defined and reported to data resources of the common access means.

The computerized profile parameters of the degree of the user's sensory processing disorders are defined based on expert knowledge. Moreover, the computerized profile parameters of the degree of the user's sensory processing disorders retrieved from unit data of a new user assigned to a value on the measurement scale are included when calculating at least one set of predefined parameters corresponding to at least one value on the defined measurement scale for a subsequent user. The at least one computerized profile parameter of the degree of the sensory processing disorder from the test is generated separately for: tactile reactivity (RO), vestibular reactivity (VeR), auditory reactivity (AR), visual reactivity (ViR), olfactory reactivity (OR), oculomotor control (OC), balance (B), praxes (P), reflex integration (IP), visual-motor coordination (VMC), distribution of antigravity muscles tension (DAMT).

The age of the users is divided into four age categories: 12-23 months; 24-35 months; 36-47 months; and 48-59 months.

When calculating the number of users, only users are counted who have a similar degree of sensory processing disorder or are assigned to the same or similar value on the measurement scale as the given user.

Preferably, when calculating the user's age, only users are counted who have an essentially similar degree of the sensory processing disorder or are assigned to the same value on the measurement scale as the given user and/or assigned to the same or similar age category.

Preferably, also information is generated about the number of users who are of the same age, who have the same value assigned on the measurement scale, and who have the same diagnostic profile assigned as the given user.

Preferably, information about the number of users, their ages, their assignment to the same value on the measurement scale, the diagnostic profile, therapy duration, and the therapy effects are stored in the common access means.

Moreover, the method comprises a step of reporting the generated set of predefined parameters corresponding to at least one value on a defined measurement scale to common access means.

Preferably, the graphical representation is an animation showing the way of performing rehabilitation exercises. Also, the graphical representation may be a 3D animation or hologram. The graphical representation is arranged on a grid of the monitor of the computer-based device associated with the user's caregiver. Preferably, the graphical representation shows the way of performing rehabilitation exercises with the use of rehabilitation tools and devices.

The step of generating the diagnostic profile also includes a step of making available the rehabilitation plan in the form of a schedule containing a guidance/list for at least one rehabilitation exercise or a set of rehabilitation exercises to be performed according to the schedule of the rehabilitation plan along with a division into days of a month and with minimum time assigned for practicing thereof for each of the days.

Preferably, the schedule is presented in the form of a diary, and it is separated from the guidance/list of the set of rehabilitation exercises, wherein after selecting a day in the diary, a set of rehabilitation exercises is displayed in an appropriate order of execution.

Preferably, after a predefined time period, a control test is generated for the given user to verify the effects of performing the set of rehabilitation exercises for the given diagnostic profile.

An update of the user's diagnostic profile is made within a private region of the common access means in the user profile assigned to the user.

### Benefits

The solution of the invention will:
- Decrease the absence from school and from work; eliminate the additional stress caused by the absence, and most of all will make the rehabilitation process significantly shorter and simpler,
- Reduce non-financial costs such as the worsening of the quality of life, decreasing the aptitude for learning, difficulties in everyday activities,
- Reduce the amount of long-term care for children unable to perform independent social functioning,
- Decrease the costs of medical, psychological, pedagogical, and logopedic care in psychological-pedagogical centers,
- Decrease the therapy costs of children with ADHD, autism, and other diseases with accompanying sensory processes disorders, by an early intervention overtaking the cumulation of disorders,
- Allow increasing self-esteem and emotional and social safety,
- Reduce the number of medical errors.

As a result, increased will be the detection of sensory disorders for the youngest children (below 4 y.o.), early diagnostics, and early intervention in different age groups (applying the rehabilitation).

### Definitions

**User** - child, up to 5 y.o., most preferably up to 4 y.o.;
SI - disorders of sensory integration
**Age categories:**
   1. Category: 12-23 months,
   2. Category: 24-35 months,
   3. Category: 36-47 months,
   4. Category: 48-59 months.
**User's caregiver** - legal guardian, physician, therapist, SI therapist; interchangeably with caregiver;
**Reconnaissance** - includes questions related to the user, such as the reason for applying for the examination, medical diagnosis, the course of pregnancy, delivery, newborn score, decreased muscle tone, asymmetry, development of speech, administered pharmaceuticals, epilepsy, allergy, heart diseases, hypertension, atopic dermatitis, asthma, orthopedic problems, otitis, vision defect, light sensitivity, hearing defect, auditory sensitivity, sensitivity to hugging, sensitivity to twirling, sensitivity to dandling, ataxia, self-service, social communication, motion activity, play, concentration, falling asleep, eating, medical history, other therapies.
**Questionnaire** - described below in the part Detailed Analysis
**Test of sensory processing disorder** - also referred to as the test; a test performed by a child with the assistance of the user's caregiver; the test is divided into tasks checking the degree of disorders of the sensory modulation and the degree of the motor disorder of a sensory basis; the test is performed with the use of a set of aids for tests by the user's caregiver; the test consists of tasks verifying the degree of disorders:
   i) tactile reactivity
      ⇒ superficial sensation
      ⇒ deep sensibility and proprioception
   ii) vestibular reactivity (VeR),
      ⇒ rotary motion
      ⇒ linear motion, variations of the body position in the space,
   iii) auditory reactivity (AR),
      ⇒ sounds produced with instruments
      ⇒ sounds reproduced,
   iv) visual reactivity (ViR),
   v) olfactory reactivity (OR),
   vi) oculomotor control (OC),
   vii)balance (B),
   viii) praxes (P),
   ix) reflex integration (RI),
   x) visual-motor coordination (VMC),
   xi) distribution of antigravity muscles tension (DAMT);
   Based on the task performed by the user, the caregiver assesses the user's behavior when doing each task separately, using a score scale - a degree of his/her sensitivity or a degree of avoidance; for each of the disorders from i) to xi) the user's sensitivity degree is computed and/or the user's avoidance degree based on the gained score;
**Point scale** - the user's behavior during every task of the test is assessed in a point scale P 1 2 3 4 5 6 7 8 N, where P denotes a hyposensitivity degree and N denotes a hypersensitivity degree.
**Measurement scale** - points reflecting a degree of the sensory processing disorder from i) to xi) are assigned to the measurement scale; the measurement scale is constituted by groups of children of a given degree of the sensory processing disorder from i) to xi), defined by expert knowledge,
**Value on the measurement scale** - one group of children of a given degree of the sensory processing disorder from i) to xi); to a given value on the measurement scale assigned are points of every sensory processing disorder from i) to xi) and/or diagnostic profile, also: a profile of sensory processing disorders;
**Set of predefined parameters** - a set of reference/control data that includes parameters of the degree of the sensory processing disorder from i) to xi) along with their assignment to a relevant value on the measurement scale, wherein for every new user databases are searched for a set of predefined parameters closest to the set of computerized profile parameters of the new user; the set of computerized of profile parameters of the new user, after its assignment to a relevant value on the measurement scale, is added to data resources of the set of predefined parameters;
**Set of computerized user profile parameters** - a set of parameters of a degree of sensory processing disorder with a division into disorders from i) to xi) from the set of predefined parameters and generated for a given user based on the questionnaire and the test of the sensory processing disorder.
**User profile** - comprising a user's data: personal data, a degree of sensory processing disorder, a profile of sensory processing disorders, a diagnosis, a set of computerized user profile parameters, a set of predefined parameters generated for the given user, a value of the measurement scale of sensory processing disorders to which the given user has been assigned, a diagnostic profile.
**Diagnostic profile** (DP, also a profile of sensory processing disorders) - assignment of a user to a given value on the measurement scale of sensory processing disorders and to the rehabilitation plan assigned thereto.
**Rehabilitation plan** - includes a plan generated for a given diagnostic profile, including at least one rehabilitation exercise or a set of rehabilitation exercises to be performed for a defined time period.
**Diagnostic tool/device** - devices intended to make it possible to perform a rehabilitation exercise, for example, barrels, movable chairs, cradles, balance beams, slides, calenders, skateboards, mattresses, tables, curtains, and any other rehabilitation sets.
**Reference data** - also, interchangeably, control data.

### Detailed embodiment

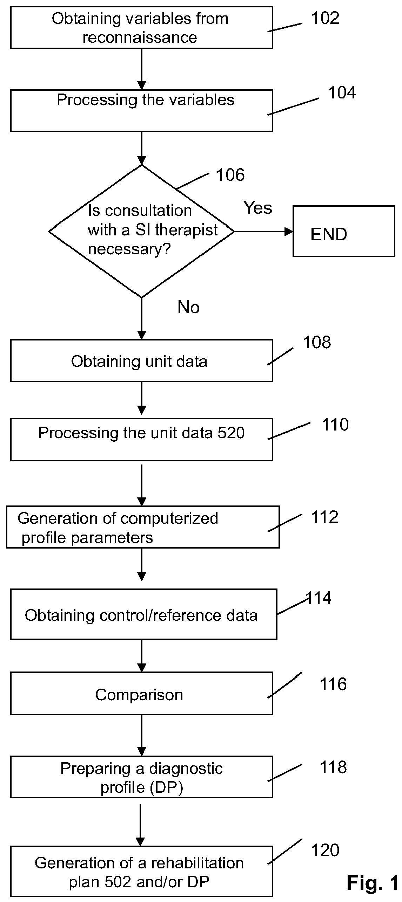

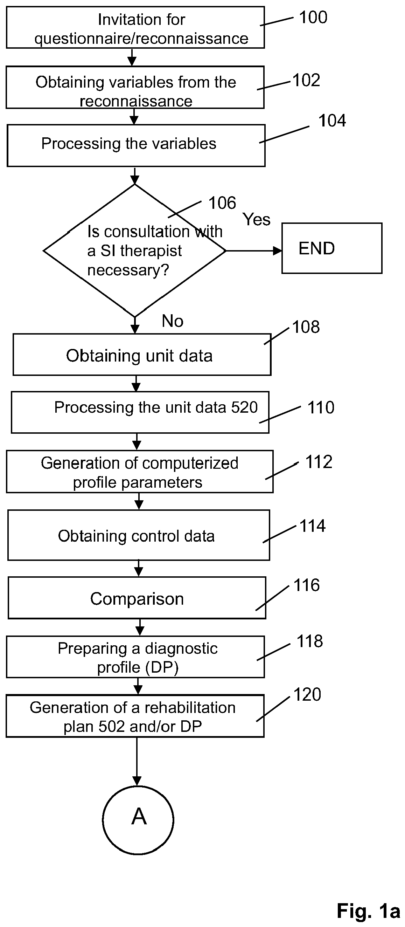

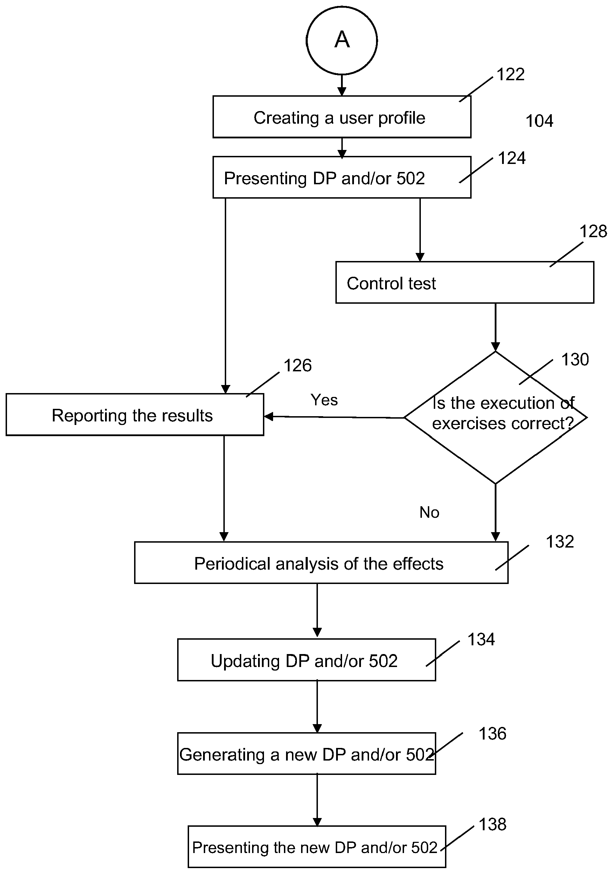

Fig. 1 shows an embodiment of a system for generating a user's rehabilitation plan, including steps of assigning the user to a diagnostic profile assigned to a defined measurement scale of sensory processing disorders, communicating to a computer network with the use of computer-based devices, 402-408, and accessing data, 416, 418 via access means, 414. In this system, a reconnaissance with the user's caregiver is carried out in order to obtain, 102, variables describing behavioral features of the user retrieved from the reconnaissance. Next, they are processed, 104, in order to qualify, 106, the user for further diagnostic tests and/or a consultation with a sensory integration therapist. If the disorders are significantly above the average, the user's caregiver obtains a recommendation to consult with an SI therapist or a therapist for sensory integration disorders. If the disorders are compliant with defined profiles, then a procedure is continued to generate a user's rehabilitation plan comprising an assignment of the user to a diagnostic profile assigned to a defined measurement scale of sensory processing disorders.

Next, unit data is obtained, 108, that includes data related to the degree of the sensory processing disorder:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii)balance (B),
viii) praxes (P),
ix) reflex integration (RI),
x) visual-motor coordination (VMC),
xi) distribution of antigravity muscles tension (DAMT),

The unit data comprises the user's data retrieved from:
- a Test of Sensory Processing Disorders, and/or
- a questionnaire.

Then, the data is processed, 110, in order to generate, 112, at least one set of computerized profile parameters of the degree of the user's sensory processing disorders. After obtaining, 114, the reference/control data comprising at least one set of predefined parameters corresponding to at least one defined measurement scale, the at least one set of computerized profile parameters is compared, 116, with the at least one set of predefined parameters corresponding to at least one measurement scale in order to establish a degree of the user's sensory processing disorder.

The unit data includes data related to the degree of sensory processing disorder:
xii)tactile reactivity (TR),
xiii) vestibular reactivity (VeR),
xiv) auditory reactivity (AR),
xv)visual reactivity (ViR),
xvi) olfactory reactivity (OR),
xvii) oculomotor control (OC),
xviii) balance (B),
xix) praxes (P),
xx)reflex integration (RI),
xxi) visual-motor coordination (VMC),
xxii) distribution of antigravity muscles tension (DAMT).

Next, on this basis, the diagnostic profile corresponding to at least one value on the measurement scale is assigned, 118, to at least one set of computerized user profile parameters, and a rehabilitation plan, 502, is generated, 120, for the user assigned to the given diagnostic profile. The rehabilitation plan, 502, is generated using a computer-based device, 402-408.

Fig. 1a shows a second embodiment of the system for generating a user's rehabilitation plan, 502, comprising a step of assigning the user to a diagnostic profile assigned to a defined measurement scale of sensory processing disorders, wherein common access means, 414, sends, via a network, an electronic invitation, 100, to a computer-based device, 402-408, associated with the user to perform a user electronic questionnaire/reconnaissance, wherein every question of the user electronic questionnaire/reconnaissance is configured to measure behavioral features of the user and to assign them to the defined measurement scale of sensory processing disorders. In response to the invitation, 100, a reconnaissance is performed with the user's caregiver in order to obtain, 102, variables describing the behavioral features of the user retrieved from the reconnaissance. Next, they are processed, 104, in order to qualify, 106, the user for further diagnostic tests and/or a consultation with a sensory integration therapist. If the disorders are significantly above the average, the user's caregiver obtains a recommendation to consult with an SI therapist. If the disorders are compliant with defined profiles, then a procedure is continued to generate a user's rehabilitation plan comprising an assignment of the user to a diagnostic profile assigned to a defined measurement scale of sensory processing disorders. Next, unit data is obtained, 108, comprising user's data retrieved from:
iii) a Test of Sensory Processing Disorders,
iv) a questionnaire.
Then, the data is processed, 110, in order to generate, 112, at least one set of computerized profile parameters of the degree of the user's sensory processing disorders. After obtaining, 114, the reference/control data comprising at least one set of predefined parameters corresponding to at least one defined measurement scale, the at least one set of computerized user profile parameters is compared, 116, with at least one set of predefined parameters corresponding to the at least one measurement scale in order to establish a degree of the user's sensory processing disorder.

Next, on this basis, the diagnostic profile corresponding to at least one value on the measurement scale is assigned, 118, to at least one set of computerized profile parameters, and a rehabilitation plan, 502, is generated, 120, for the user assigned to the given diagnostic profile. The rehabilitation plan, 502, is generated using a computer-based device, 402-408.

Then, for the given diagnostic profile and the user's rehabilitation plan, 502, a user profile is generated, 122. According to the invention, the user profile includes at least one element from the following: personal data, a degree of sensory processing disorder, a diagnosis, a set of computerized user profile parameters, a set of predefined parameters generated for the given user, a value of the measurement scale to which the given user is assigned, a diagnostic profile. According to the invention, a user ID identifier is assigned to the user profile. Moreover, the user profile may be created with the use of predefined elements of user profiles. The user profile is created, 122, and stored on a server, wherein the server is a part of the common access means, 414. The rehabilitation plan, 502, for the given diagnostic profile in the form of at least one rehabilitation exercise is presented, 124, to the user using a computer-based device 402-408 associated with the user, in the form of graphical representations, 500. The rehabilitation plan, 502, comprising the assignment of the user to the given diagnostic profile and assigned thereto effects of performing at least one exercise or at least one set of rehabilitation exercises along with an assigned thereto schedule, 504, is periodically reported, 126, to common access means, 414, in the form of a computer file containing the user ID identifier. Also, it is possible that after a predefined time period, a control test is generated, 128, verifying the effects of performing the at least one exercise or at least one set of rehabilitation exercises of the given diagnostic profile assigned to the given user. It is possible that in the rehabilitation plan, 502, the user has planned at least one exercise or a set of exercises for only one disorder and/or any number of mentioned disorders: tactile reactivity (TR), vestibular reactivity (VeR), auditory reactivity (AR), visual reactivity (ViR), olfactory reactivity (OR), oculomotor control (OC), balance (B), praxes (P), reflex integration (RI), visual-motor coordination (VMC), distribution of antigravity muscles tension (DAMT). If the exercise and/or exercises listed in the rehabilitation plan, 502, is/are performed, 130, correctly, the effects of performing the exercise and/or rehabilitation exercises along with the assigned thereto rehabilitation plan are periodically reported, 126, to the common access means, 414, in the form of a computer file containing the user ID identifier. If the exercise or exercises are performed, 130, incorrectly, and also after obtaining reported, 126, effects of performing the rehabilitation exercise and/or exercises along with the assigned thereto rehabilitation plan, 502, a next step may follow, namely a step of analyzing, 132, the reported rehabilitation plans, 502, the diagnostic profiles, including the effects of performing the exercise and/or the set of rehabilitation exercises, and/or the results of the user control test, wherein the effects of the rehabilitation progress of users belonging to the same age category are grouped into several groups assigned to the same or a similar value on the measurement scale and/or to a given diagnostic profile. The correctness of execution, 130, of the rehabilitation exercise and/or exercises is assessed according to a predefined point scale. Next, the results of the analysis, 132, are transferred to an update step, 134, for updating the rehabilitation plan, 502, and/or the diagnostic profile, which is executed in databases of the common access means, 414, and is transferred by the common access means, 414, in the form of a computer file containing the user ID identifier to the user's computer-based device, 402-408. The update, 134, of the rehabilitation plan, 502, and/or the diagnostic profile is based on the following:
- assigning the user to another value on the measurement scale, and/or
- assigning the user to another diagnostic profile, and/or
- assigning the rehabilitation exercise and/or exercises to a new value on the measurement scale of sensory processing disorders, and/or
- adding at least one new rehabilitation exercise and/or at least one set of rehabilitation exercises to the given diagnostic profile.
After updating, 134, a new diagnostic profile is generated, 136, and a new rehabilitation plan, 502, is assigned thereto along with at least one rehabilitation exercise and/or at least one set of rehabilitation exercises and/or extending the execution time of this diagnostic profile. After generating, 136, the new diagnostic profile, the new diagnostic profile, along with the new rehabilitation plan, 502, is presented to the user.

The generated diagnostic profile includes rehabilitation exercises for at least one of the sensory processing disorders:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii)balance (B),
viii) praxes (P),
ix) reflex integration (RI),
x) visual-motor coordination (VMC),
xi) distribution of antigravity muscles tension (DAMT),

According to the invention, the diagnostic profile with an assigned thereto rehabilitation plan, 502, corresponds to the user age category and is generated for a defined time period, preferably for 4 weeks.

According to the invention, the reference/control data consists of at least one set of predefined parameters assigned to a given value of the measurement scale that is predefined and delivered by the common access means.

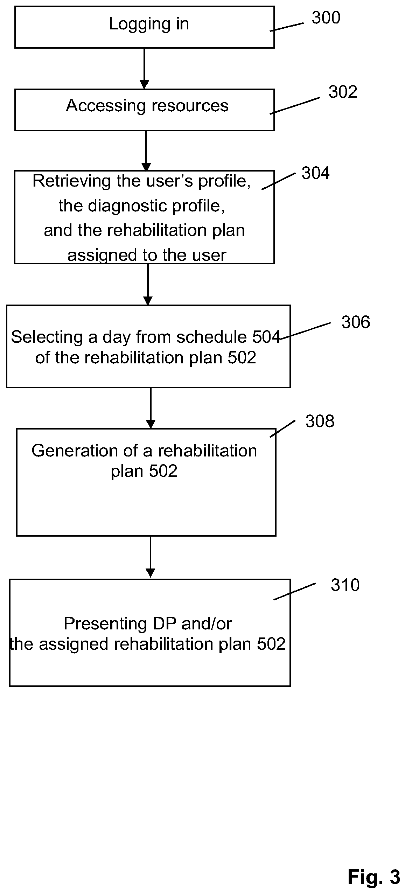

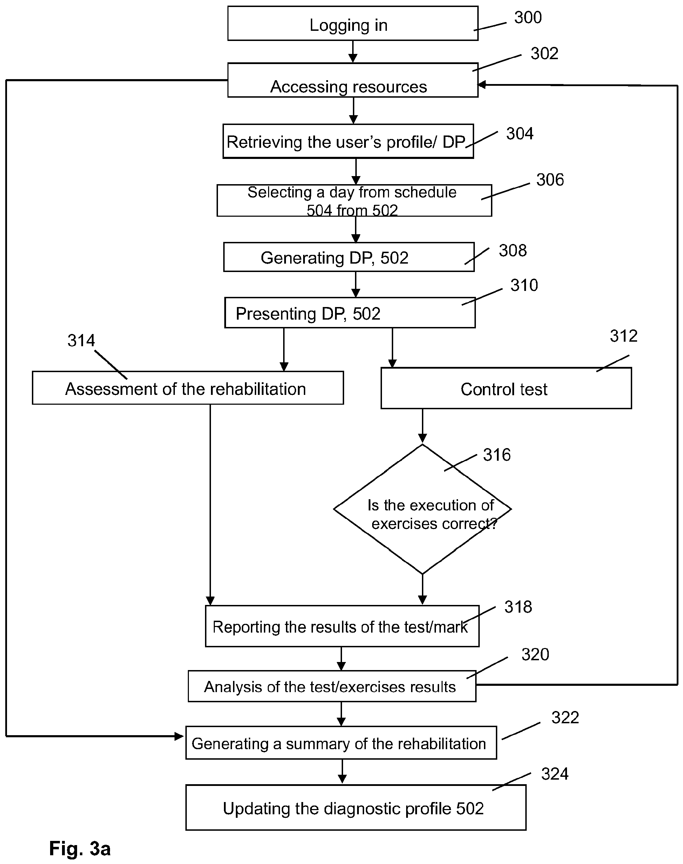

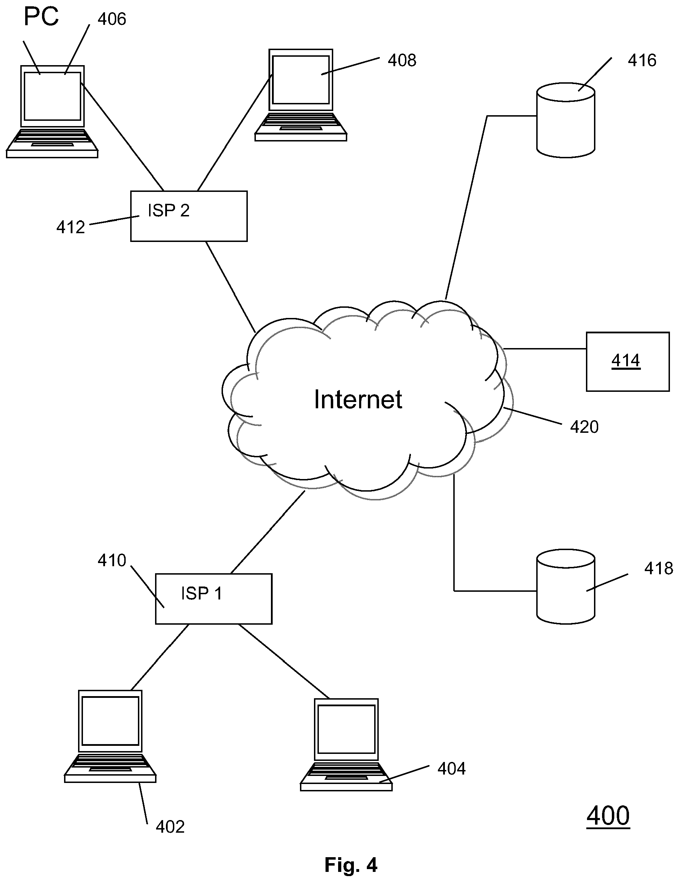

Figs. 3 and 4 show one embodiment of a method for generating and presenting a diagnostic profile to a user with the use of resources of computer data, 416, 418. In this embodiment, caregivers of the users communicate with a computer network, 400, with the use of computer-based devices, 402 - 408. In this particular embodiment illustrating the Internet, the connection is established by means of access apparatus of Internet service providers (ISP), 410 and 412, wherein access equipment is a part of the network infrastructure, using, for example, xDSL links or any other accessible service. The function of the ISP provider is to create a physical connection of subscriber computers 402 - 408 to the Internet. However, it is possible to connect the computer-based devices 402 - 408 mutually in the form of a local network (LAN), wherein this local network LAN is connected to the Internet by means of a leased link. The users' caregivers access, in step 302, to data resources, 416, 418 by means of common access means, 414. The common access means, 414, in a simple embodiment, is a server controlled by software in such a way that it acts as a website for the user's caregiver. In more complex embodiments, when a huge network has to be managed, it may be a group of servers controlled by such software. In order to take advantage of the services offered by the common access means, 414, a user's caregiver proceeds to a logging process in step 300, in which an identification and authorization procedure is executed.

In other embodiments, one may be serviced by the common access means, 414, without the logging process.

After being logged in step 300, the user's caregiver enters an assigned private region. In the common access means, 414, data is stored related to the given user. Next, in step 302, the user's caregiver accesses the data resources and downloads, 304, the user profile and/or the diagnostic profile, and/or the rehabilitation plan, 502. the user profile has stored, among others, the user's diagnosis, the rehabilitation plan, 502, control test results, as well as the user's progress in performing diagnostic exercises (also rehabilitation exercises). To the user profile, a diagnostic profile is assigned that includes a rehabilitation plan, 502, assigned thereto and relevant exercise and/or rehabilitation exercises with specific times indicated for practicing. After selecting, 306, a day from the schedule, 504, a rehabilitation plan, 502, for the given day is generated, 308, showing at least one exercise or a set of rehabilitation exercises the user should execute, also showing the order and the practicing time for every exercise of the set of rehabilitation exercises. Next, at least one exercise and/or at least one set of rehabilitation exercises is presented, 310, in the form of a graphical representation, 500, arranged, for example, on the grid 550 of the screen of the computer-based device, 402-408, associated with the user's caregiver.

In one embodiment, a diagnostic profile or a rehabilitation plan, 502, may be presented, 310. The rehabilitation plan may be presented in the form of a schedule, 504, including a guidance/list, 506, of rehabilitation exercises to be performed according to the schedule of the rehabilitation plan along with a division into days, 508, of a month and with a minimum time of practicing assigned to every day. In the assigned user profile, the user's caregiver arbitrarily selects desired information: the assignment of the user to a new/old diagnostic profile and/or rehabilitation plan, 502, and/or exercises to be performed on the given day or within a month.

According to the invention, the rehabilitation plan, 502, assigned to a user's diagnostic profile and presented, 310, on the grid 550 of the screen of the computer-based device, 402-408, contains at least one element of the following: a schedule, 504, a guidance/list, 506, an exercise and/or a set of rehabilitation exercises to be performed according to the schedule of the rehabilitation plan, days, 508, of a month of the rehabilitation plan or a graphical representation, 500, of the rehabilitation exercise. This is data that may be downloaded directly from the data resource and is provided by the owner of the data resource, 416, 418.

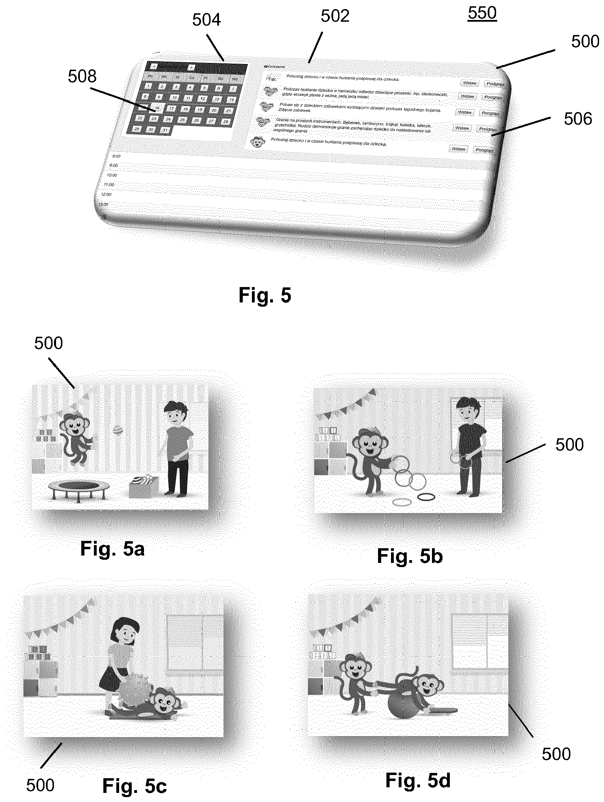

For the user, it is very important how the graphical representation, 500, is presented. Figures 5a-5d show a screen presenting the rehabilitation exercises located in a private directory, according to one embodiment of the present invention. In this embodiment, the way of performing an exercise is shown on grid 550 of the screen of the computer-based device, 402-408, by means of a cartoon animation. The cartoon animation may feature an ape, as shown in Fig. 5a-5d, or another cartoon character that may be downloaded via the common access means, 414, from the data resources. Also, the rehabilitation exercises may illustrate the way how the caregiver should practice with the user.

In one embodiment, it is possible that a holographic device is attached to the computer-based device, 402-408, that displays a graphical representation, 500, in the form of a hologram or a 3D cartoon animation.

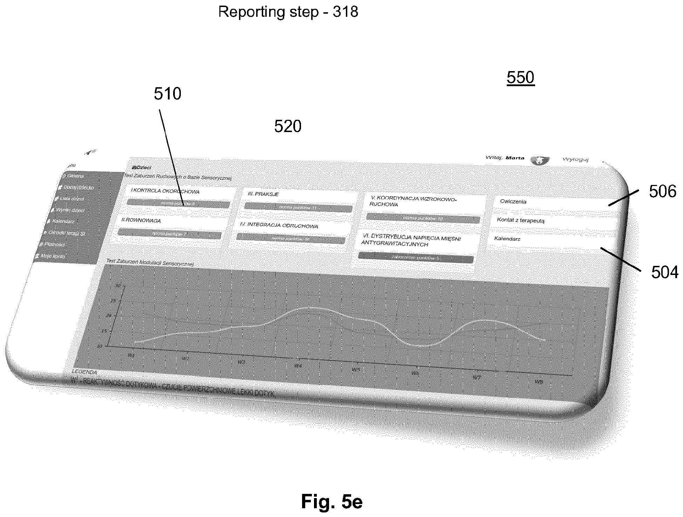

In another embodiment shown in Fig. 5e, on the grid, 550, of the screen of the computer-based device, 402-408, a graphical representation, 500, may be shown in the form of graphically represented user's unit data, 520. The grid, 550, may display information, 510, about the degree of the sensory processing disorder for each of the disorders from i) to xi), a bookmark of the diary, 504, or a bookmark of the exercises, 506, to be performed, or graphs showing the recent effects of practiced exercises or a change of the diagnostic profile.

Fig. 3a shows additional further possible steps of a method for generating and presenting, to a user, a rehabilitation plan, 502 assigned to the user's diagnostic profile. According to the invention, the user undergoes a control test, 312, during which a set of control exercises to be performed by the user is displayed on the grid, 550, of the screen of the computer-based device, 402-408. The caregiver assesses the user's behavior and/or the correctness of performing an exercise on a point scale.

Also, it is possible that the user is not performing the exercises of the control test correctly because, for example, the child has a tummy ache, feels bad, is ill or fractious, etc. Then, the caregiver decides, 316, if the control test is continued or finished on such a day.

If the exercises are performed, 316, correctly, then the results of the control test are reported, 318, to the common access means, 414, in the form of a computer file or files (when sent separately) along with the user ID identifier in order to preserve the relationship of the user profile with its results of the control test and behavior.

The user's caregiver may evaluate, 314, the correctness of the at least one exercise and/or at least one set of rehabilitation exercises performed by the user every day of exercises. The marks, 314, are created in the user's private region (directory) within the common access means, 414, and thereby are accessible, 302, for processing by the common access means, 414, immediately after being created.

In one embodiment, the files with the results of the control test and/or marks of performing the set of rehabilitation exercises are reported, 318, to the common access means, 414, in real-time; however, it is also possible to send them periodically. Next, the results of the control test and/or the marks of performing the at least one exercise and/or at least one set of rehabilitation exercises are analyzed, 320, in order to establish if the child, according to the given value on the measurement scale and for the assigned diagnostic profile, performs the exercise and/or exercises correctly, with an appropriate speed, what is the child's reaction, or if progress has been noticed. Results of the analysis, 320, are transferred by the common access means, 414, to the data resources 416, 418 in order to assign them to a range of options of performing the exercises by the user and the user's behavior, to assign them to the relevant diagnostic profile. Next, a rehabilitation summary is generated, 322, for the given time period. The rehabilitation summary is generated, for example, in the form of a graphical representation, as shown in Fig. 5e.

Depending on test results, exercises, or marks, an update is created, in step 324, for the rehabilitation plan, 502, assigned to the user's diagnostic profile and/or for the diagnostic profile locally on the user's computer-based device 402 - 408. However, the update may be created on the server within the private region of the common access means 414. An updated diagnostic profile, and therefore the rehabilitation plan, 502, comprises, for example:
- an assignment of at least one exercise or at least one new set of rehabilitation exercises along with a new schedule for performing thereof, or
- extending the time for performing an earlier at least one exercise or at least one set of rehabilitation exercises according to the point scale for performing thereof, along with the schedule update.

In one embodiment of the present invention, the user's caregiver may have more than one user profile. The user's caregiver may be a therapist, a specialist, a physician, or a parent. Therefore, a therapist or a physician needs as many user profiles as many patients he/she has. Then, the therapist or the physician or the specialist performs the whole assessment process with the child, starting from the reconnaissance, questionnaire, and tests. Every user profile is defined by the caregiver of the given user. Therefore, the possibility of creating more user profiles for a single user's caregiver is a great advantage.

For better precision of the monitoring process, the measurement scale is created from predefined data, for example, from:
- reference/control data consisting of at least one set of predefined parameters assigned to a given value of the measurement scale,
- a degree of the sensory processing disorder for every disorder from i) to xi),
- an age category of the child,
- a number of children with the same disorder assessment,
- the rehabilitation time,
- rehabilitation effects,
- results of the control test.

It makes it possible to limit the number of types of values created on the measurement scale, this simplifying and accelerating the data processing. Also, the user profiles and diagnostic profiles are preliminarily defined.

However, in one embodiment, if the user's unit data, 520, after generating, 218, the computerized user's profile parameters do not correspond, 220, to any value on the measurement scale, then, in step 222, a new value is created on the measurement scale, to which the computerized user's profile parameters will be assigned, 224. Next, a given value on the scale is defined, 226, and reported, 228, to the data resources 416, 418 of the common access means, 414.

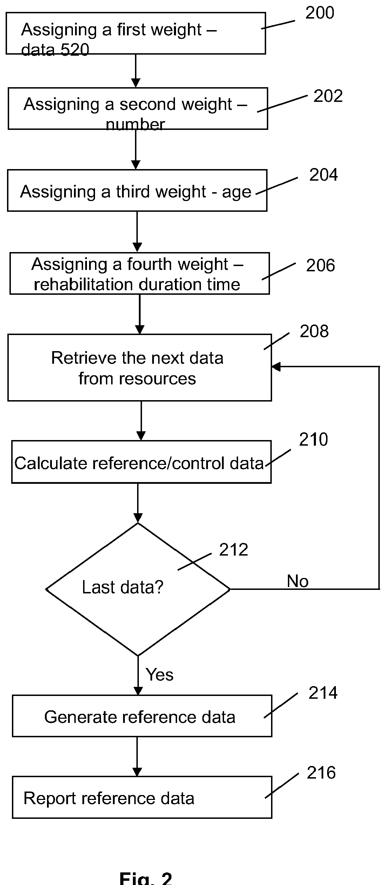

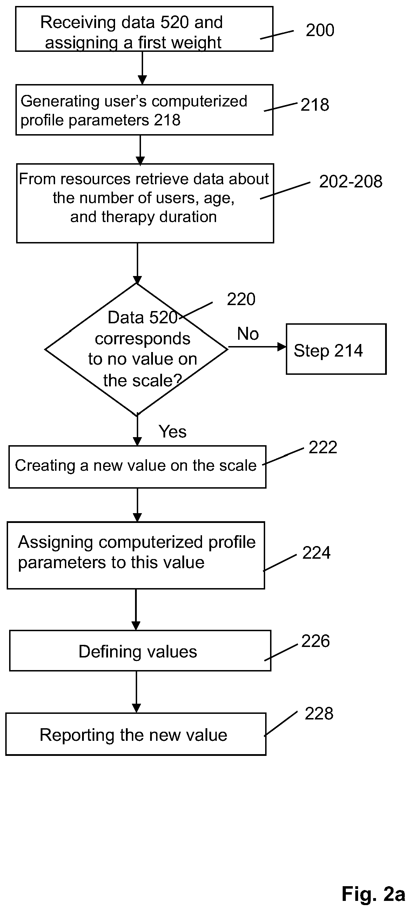

Figures 2 and 4 show an embodiment of a method of reporting the results of a search executed in a computer network to the user.

In one embodiment of the present invention related to a method for assigning profile parameters of sensory processing disorders corresponding to at least one value on a measurement (diagnostic) scale of sensory processing disorders, data is subjected to processing by the common access means, 414, in several steps. In step 200, a first weight is assigned to unitary data 520. This data is a very important factor. In the next step, 202, a second weight is assigned to the number of users who are in the same age category as the given user for which the reference/control data is calculated. In the next step, 204, a third weight is assigned to the user's age. In the next step, 206, a fourth weight is assigned to the rehabilitation duration time for the same degree of disorder of sensory perception as the user. Here, it is considered if the user was already rehabilitated in the past or if the user will be rehabilitated for the first time. Finally, in step 210, 212, reference/control data is calculated, that is, at least one set of predefined profile parameters for the given user corresponding to at least one value on a defined measurement scale based on the user unit data, the number of users, the age of users, the rehabilitation duration time, and the assigned weights.

The order of presenting the set of predefined profile parameters for the given user is defined by calculating a point mark in step, 210, for every disorder from i) to xi), i.e., tactile reactivity (TR), vestibular reactivity (VeR), auditory reactivity (AR), visual reactivity (ViR), olfactory reactivity (OR), oculomotor control (OC), balance (B), praxes (P), reflex integration (RI), visual-motor coordination (VMC), distribution of antigravity muscles tension (DAMT). In step 210, for calculating the point mark, the following are considered: the number of users of the same degree of disorders from i) to xi), the age category of users, the rehabilitation time, and the assigned weights. After calculating, in step 212, the point mark for the last data, the reference/control data is generated in step 214. In the calculation step, 210, 212, the reference data may be calculated taking into consideration only the users of essentially the same user profiles as the user profile for which the reference data is searched. Similarly, when calculating the number of users, only users are counted of essentially the same user profiles as the profiles of the given user and/or of the same degree of sensory perceiving from i) to xi) as the given user. With such exploitation of data collected in the process of assigning the reference data as defined earlier, it is possible to assign the user to the most appropriate value on the measurement scale. Next, the reference data is reported, 216, to the data resources 416, 418 of the common access means 414.

It is possible, in another embodiment, that the user profiles will not be considered when calculating the reference data and/or the users of the same degree of disorders of sensory perception from i) to xi) and/or of the same user profile will not be counted. In this embodiment, a general average is calculated.

Fig. 4 shows an embodiment of a computer network 400. For clarity, the drawings show the invention very schematically, where elements and lines that are not of essential importance for understanding the invention have been omitted. The computer network 400 contains a plurality of computer-based devices 402 - 408 connected to an access apparatus of Internet service providers (ISP) 410, 412, the access equipment being a part of the network infrastructure, and also at least common access means 414 making it possible to access the data resources 416, 418. However, it is possible that computer-based devices 402 - 408 are interconnected in the form of a local network (LAN), and the network LAN is connected to the Internet by means of a leased line. The user device 402 - 408 and the common access means 414 may be remote, and the communication between them is transferred via a network, for example, the Internet 420. The common access means 414 is adapted to cooperate with the network.

The computer-based device 402 - 408 may be a personal computer, a laptop, a cellular phone, Personal Digital Assistant, or any other microprocessor-based device adapted to cooperate with the computer network.

### Methodology for generating a user's rehabilitation plan

SI therapists carried out research work on a group of 240 children aged from 1 to 5 years in specially adapted rehabilitation rooms. When selecting patients for this research, they tried to obtain four more or less equinumerous groups in terms of age. The groups to which the patients were assigned were the following age ranges: 1 to 2 years, 2 to 3 years, 3 to 4 years, and 4 to 5 years. Moreover, equal numbers of patients with SI disorders and patients with no such disorders have been selected for each of the above-mentioned groups. Other criteria for selecting children for the research were random.

Data were provided by the researchers in the form of databases being the result of carried out reconnaissance, test, questionnaire, and observation. Every patient was described by a set of features, including both features of nominal values as well as numerical values. Among the features describing the user, metadata has been included coming from the reconnaissance, such as the age, medical history, and data related to the test and questionnaire results.

### Research tools

The research tools included:
- Basic Reconnaissance
- Test of Sensory Processing Disorders for users aged from 1 to 2 years and from 3 to 4 years.
- Questionnaire of Sensory Development for users' parents.

### Basic Reconnaissance

The Basic Reconnaissance consisted of 19 questions related to the course of pregnancy, child's diseases, and questions about the activity.

### Test of Sensory Processing Disorders

The Test of Sensory Processing Disorders consisted of questions creating:
Parts of the Test of Sensory Modulation Disorders, which comprised eight indicators defined on a scale: avoiding and sensitivity.
TACTILE REACTIVITY - superficial sensation, delicate touch - Sensitivity
TACTILE REACTIVITY - superficial sensation, delicate touch - Avoiding
TACTILE REACTIVITY - deep sensibility, touch with pressure, and proprioception - Sensitivity
TACTILE REACTIVITY - deep sensibility, touch with pressure, and proprioception - Avoiding
VESTIBULAR REACTIVITY - rotary motion - Sensitivity
VESTIBULAR REACTIVITY - rotary motion - Avoiding
VESTIBULAR REACTIVITY - linear motion, variations of the body position in the space - Sensitivity
VESTIBULAR REACTIVITY - linear motion, variations of the body position in the space - Avoiding
AUDITORY REACTIVITY - sounds produced with instruments - Sensitivity
AUDITORY REACTIVITY - sounds produced with instruments - Avoiding
AUDITORY REACTIVITY- sounds reproduced - Sensitivity
AUDITORY REACTIVITY- sounds reproduced - Avoiding
VISUAL REACTIVITY - Sensitivity
VISUAL REACTIVITY - Avoiding
OLFACTORY REACTIVITY - Sensitivity
OLFACTORY REACTIVITY - Avoiding

And parts of the Test of Motor Disorders with Sensory Basis, which comprised six indicators.
OCULOMOTOR CONTROL
BALANCE
PRAXES
REFLEX INTEGRATION
VISUAL-MOTOR COORDINATION
DISTRIBUTION OF ANTIGRAVITY MUSCLES TENSION

### Questionnaire of sensory development

The Questionnaire on sensory development consisted of questions creating the following scales:
Tactile hypersensitivity
Vestibular hypersensitivity
Auditory hypersensitivity
Olfactory hypersensitivity
Visual hypersensitivity
Decreased muscle tone
Auditory hyposensitivity
Deep sensibility, hyposensitivity, proprioception
Olfactory hyposensitivity
Visual hyposensitivity
Visual-motor coordination disorders
Balance disorders
Vestibular hyposensitivity.

### Variables

Variables included in the Test of Sensory Processing Disorders have been created based on summing the results of responses to questions comprised in the given scale.

### Variables included in the questionnaire

Questions have been re-coded that are included in reversed scales - of a negative connotation (0 = 10; 1=9; 8=2, etc.).

Because of missing answers to some questions included in the variables, the arithmetic average has been employed when creating the indicator.

In this way, 24 variables were created. The results of the variables were summed, thus creating indicators.

In this way, 13 indicators were obtained.

### Mapping of scales

Below, the questionnaire scales and corresponding test scales are presented.

| **Mapping of test scales with questionnaire scales** | | | | | | | |
|---|---|---|---|---|---|---|---|
| Lp | **Questionnair e scales** | **scales of the Test of Sensory Modulation Disorders** | | **Scales of the Test of Motor Disorders with Sensory Basis** | | | |
| 1 | **Tactile hypersensitiv ity** | TACTILE REACTIVIT Y-superficial sensation, delicate touch | TACTILE REACTIVIT Y - deep sensibility, touch with pressure, and proprioceptio n | | | | |
| 2 | **Vestibular hypersensitiv ity** | VESTIBUL AR REACTIVIT Y - rotary motion | VESTIBUL AR REACTIVIT Y - linear motion, variations of the body position in the space | | | | |
| 3 | **Auditory hypersensitiv ity** | AUDITORY REACTIVIT Y - sounds produced with instruments | AUDITORY REACTIVIT Y- sounds reproduced | | | | |
| 4 | **Olfactory hypersensitiv ity** | OLFACTOR Y REACTIVIT Y | | | | | |
| 5 | **Visual hypersensitiv ity** | VISUAL REACTIVIT Y | | | | | |
| 6 | **Decreased muscle tone** | VESTIBUL AR REACTIVIT Y - rotary motion | VESTIBUL AR REACTIVIT Y - linear motion, variations of the body position in the space | BALANCE | DISTRIBUTI ON OF ANTIGRAVI TY MUSCLES TENSION | REFLEX INTEGRATI ON | OCULOMOT OR CONTROL |
| 7 | **Auditory hyposensitivi ty** | AUDITORY REACTIVIT Y - sounds produced with instruments | AUDITORY REACTIVIT Y- sounds reproduced | | | | |
| 8 | **Deep sensibility, hyposensitivi ty, proprioceptio n** | TACTILE REACTIVIT Y - deep sensibility, touch with pressure, and proprioceptio n | | | | | |
| 9 | **Olfactory hyposensitivi ty** | OLFACTOR Y REACTIVIT Y | | | | | |
| 10 | **Visual hyposensitivi ty** | VISUAL REACTIVIT Y | | | | | |
| 11 | **Disorders of visual-motor coordination** | | | VISUAL-MOTOR COORDINATI ON | | | |
| 12 | **Balance disorders** | VESTIBUL AR REACTIVIT Y - rotary motion | VESTIBUL AR REACTIVIT Y - linear motion, variations of the body position in the space | BALANCE | DISTRIBUTI ON OF ANTIGRAVI TY MUSCLES TENSION | REFLEX INTEGRATI ON | OCULOMOT OR CONTROL |
| 13 | **Vestibular hyposensitivi ty** | VESTIBUL AR REACTIVIT Y - rotary motion | VESTIBUL AR REACTIVIT Y - linear motion, variations of the body position in the space | BALANCE | DISTRIBUTI ON OF ANTIGRAVI TY MUSCLES TENSION | REFLEX INTEGRATI ON | OCULOMOT OR CONTROL |
| | | | | PRAXES | POSTURAL PRAXES | MANUAL PRAXES | PRAXES ORALNE |

### Analysis

### Sample

In the research, 240 children with parents were engaged. 59 patients (25.1%) were children aged from 1 to 2 y.o., 56 patients (23.7%) were children in the age group from 2 to 3 y.o., 69 patients (29.1%) were children in the age group from 3 to 4 y.o., 45 children (18.9%) were at the age of 4 to 5 years. In the research, SI disorders were diagnosed for 117 patients (48.7%) and no SI disorders for 112 patients (46.8%).

### Analysis of distributions

First, distributions of all analyzed variables were analyzed.

The analysis showed that a vast majority of variables do not have a distribution similar to the normal distribution. Because of the studied sample of n>10, further research was carried out based on parametric tests.

For every variable, descriptive statistics have been calculated - measures of the central tendency.

### Comparison of groups

In the next step, compared were the results of the examined children from the groups with disorders and without disorders in terms of the test and questionnaire scales.

The analysis showed that the differences between the groups are statistically significant (p<0.05) in terms of the majority of the variables. Next, a multi-factor analysis of variance was carried out.

As a multi-factor analysis, one considers such an analysis in which at least two independent variables (classifying factors) are involved. In this case, the classifying factors were the age range and the group with disorders vs. children without disorders.

The analysis showed that the influence of the age group on the results of the patients was small. Only for the variables:
VESTIBULAR REACTIVITY - rotary motion - Sensitivity
VESTIBULAR REACTIVITY - rotary motion - Avoiding
A statistically significant influence was observed for the age group p<0.05.

On the contrary, the influence on the variables of the groups with disorders and without disorders was completely different.

A statistically significant influence on practically all analyzed variables, besides (p>0.05), was observed for the factor: group with disorders and without disorders.
VISUAL REACTIVITY - Sensitivity
OLFACTORY REACTIVITY - Sensitivity

In the case of the interaction of two factors simultaneously (the age group and the group with disorders and without disorders), statistically significant results were only observed for variables (p<0.05)
TACTILE REACTIVITY - deep sensibility, touch with pressure, and proprioception - Sensitivity
TACTILE REACTIVITY - deep sensibility, touch with pressure, and proprioception - Avoiding

### Analysis of relations between variables.

In the research, relations between all analyzed variables were verified.

### Results of the analysis of correlations

### Correlations within test indicators

Statistically significant relations are observed between OCULOMOTOR CONTROL and TACTILE REACTIVITY - superficial sensation, delicate touch - Avoiding r=-0.16 p<0.05. With an increase of OCULOMOTOR CONTROL, TACTILE REACTIVITY - superficial sensation, delicate touch - Avoiding is decreasing
Statistically significant relations are observed between OCULOMOTOR CONTROL and TACTILE REACTIVITY - deep sensibility, touch with pressure, and proprioception - Sensitivity r=-0.14 p<0.05. With an increase of OCULOMOTOR CONTROL, TACTILE REACTIVITY - deep sensibility, touch with pressure, and proprioception - Sensitivity is decreasing
Statistically significant relations are observed between OCULOMOTOR CONTROL and VESTIBULAR REACTIVITY - linear motion, variations of the body position in the space - Sensitivity r=-0.13 p<0.05. With an increase of OCULOMOTOR CONTROL, VESTIBULAR REACTIVITY - linear motion, variations of the body position in the space - Sensitivity is decreasing
Statistically significant relations are observed between OCULOMOTOR CONTROL and VESTIBULAR REACTIVITY - linear motion, variations of the body position in the space - Avoiding r=-0.13 p<0.05. With an increase of OCULOMOTOR CONTROL, VESTIBULAR REACTIVITY - linear motion, variations of the body position in the space - Avoiding is decreasing
Statistically significant relations are observed between OCULOMOTOR CONTROL and OLFACTORY REACTIVITY - Sensitivity r=-0.15 p<0.05. With an increase of OCULOMOTOR CONTROL, OLFACTORY REACTIVITY - Sensitivity is decreasing
Statistically significant relations are observed between OCULOMOTOR CONTROL and OLFACTORY REACTIVITY - Avoiding r=-0.14 p<0.05. With an increase of OCULOMOTOR CONTROL, OLFACTORY REACTIVITY - Avoiding is decreasing
Statistically significant relations are observed between BALANCE and TACTILE REACTIVITY - superficial sensation, delicate touch - Sensitivity r=-0.4 p<0.05. With an increase of BALANCE, TACTILE REACTIVITY - superficial sensation, delicate touch - Sensitivity is decreasing
Statistically significant relations are observed between BALANCE and TACTILE REACTIVITY - superficial sensation, delicate touch - Avoiding r=-0.36 p<0.05. With an increase of BALANCE, TACTILE REACTIVITY - superficial sensation, delicate touch - Avoiding is decreasing
Statistically significant relations are observed between BALANCE and AUDITORY REACTIVITY- sounds reproduced - Avoiding r=-0.14 p<0.05. With an increase of BALANCE, AUDITORY REACTIVITY- sounds reproduced - Avoiding is decreasing
Statistically significant relations are observed between PRAXES and TACTILE REACTIVITY - superficial sensation, delicate touch - Sensitivity r=-0.23 p<0.05. With an increase of PRAXES, TACTILE REACTIVITY - superficial sensation, delicate touch - Sensitivity is decreasing
Statistically significant relations are observed between PRAXES and TACTILE REACTIVITY - superficial sensation, delicate touch - Avoiding r=-0.22 p<0.05. With an increase of PRAXES, TACTILE REACTIVITY - superficial sensation, delicate touch - Avoiding is decreasing
Statistically significant relations are observed between PRAXES and VESTIBULAR REACTIVITY - rotary motion - Sensitivity r=-0.18 p<0.05. With an increase of PRAXES, VESTIBULAR REACTIVITY - rotary motion - Sensitivity is decreasing
Statistically significant relations are observed between PRAXES and VESTIBULAR REACTIVITY- rotary motion - Avoiding r=-0.16 p<0.05. With an increase of PRAXES, VESTIBULAR REACTIVITY - rotary motion - Avoiding is decreasing
Statistically significant relations are observed between MANUAL PRAXES and TACTILE REACTIVITY - superficial sensation, delicate touch - Avoiding r=-0.19 p<0.05. With an increase of MANUAL PRAXES, TACTILE REACTIVITY - superficial sensation, delicate touch - Avoiding is decreasing
Statistically significant relations are observed between MANUAL PRAXES and TACTILE REACTIVITY - deep sensibility, touch with pressure, and proprioception - Sensitivity r=0.25 p<0.05. With an increase of MANUAL PRAXES, TACTILE REACTIVITY - deep sensibility, touch with pressure, and proprioception - Sensitivity is increasing
Statistically significant relations are observed between MANUAL PRAXES and TACTILE REACTIVITY - deep sensibility, touch with pressure, and proprioception - Avoiding r=0.23 p<0.05. With an increase of MANUAL PRAXES, TACTILE REACTIVITY - deep sensibility, touch with pressure, and proprioception - Avoiding is increasing
Statistically significant relations are observed between MANUAL PRAXES and OLFACTORY REACTIVITY - Sensitivity r=-0.19 p<0.05. With an increase of MANUAL PRAXES, OLFACTORY REACTIVITY - Sensitivity is decreasing
Statistically significant relations are observed between MANUAL PRAXES and OLFACTORY REACTIVITY - Avoiding r=-0.21 p<0.05. With an increase of MANUAL PRAXES, OLFACTORY REACTIVITY - Avoiding is decreasing
Statistically significant relations are observed between POSTURAL PRAXES and TACTILE REACTIVITY - deep sensibility, touch with pressure, and proprioception - Sensitivity r=0.24 p<0.05. With an increase of POSTURAL PRAXES, TACTILE REACTIVITY - deep sensibility, touch with pressure, and proprioception - Sensitivity is increasing
Statistically significant relations are observed between POSTURAL PRAXES and TACTILE REACTIVITY - deep sensibility, touch with pressure, and proprioception - Avoiding r=0.21 p<0.05. With an increase of POSTURAL PRAXES, TACTILE REACTIVITY - deep sensibility, touch with pressure, and proprioception - Avoiding is increasing
Statistically significant relations are observed between POSTURAL PRAXES and OLFACTORY REACTIVITY - Avoiding r=-0.19 p<0.05. With an increase of POSTURAL PRAXES, OLFACTORY REACTIVITY - Avoiding is decreasing
Statistically significant relations are observed between REFLEX INTEGRATION and TACTILE REACTIVITY - superficial sensation, delicate touch - Sensitivity r=-0.33 p<0.05. With an increase of REFLEX INTEGRATION, TACTILE REACTIVITY - superficial sensation, delicate touch - Sensitivity is decreasing
Statistically significant relations are observed between REFLEX INTEGRATION and TACTILE REACTIVITY - superficial sensation, delicate touch - Avoiding r=-0.31 p<0.05. With an increase of REFLEX INTEGRATION, TACTILE REACTIVITY - superficial sensation, delicate touch - Avoiding is decreasing
Statistically significant relations are observed between REFLEX INTEGRATION and OLFACTORY REACTIVITY - Sensitivity r=-0.14 p<0.05. With an increase of REFLEX INTEGRATION, OLFACTORY REACTIVITY - Sensitivity is decreasing
Statistically significant relations are observed between VISUAL-MOTOR COORDINATION and TACTILE REACTIVITY - superficial sensation, delicate touch - Sensitivity r=-0.23 p<0.05. With an increase of VISUAL-MOTOR COORDINATION, TACTILE REACTIVITY - superficial sensation, delicate touch - Sensitivity is decreasing
Statistically significant relations are observed between VISUAL-MOTOR COORDINATION and TACTILE REACTIVITY - superficial sensation, delicate touch - Avoiding r=-0.22 p<0.05. With an increase of VISUAL-MOTOR COORDINATION, TACTILE REACTIVITY - superficial sensation, delicate touch - Avoiding is decreasing
Statistically significant relations are observed between DISTRIBUTION OF ANTIGRAVITY MUSCLES TENSION and TACTILE REACTIVITY - superficial sensation, delicate touch - Sensitivity r=-0.32 p<0.05. With an increase of DISTRIBUTION OF ANTIGRAVITY MUSCLES TENSION, TACTILE REACTIVITY - superficial sensation, delicate touch - Sensitivity is decreasing
Statistically significant relations are observed between DISTRIBUTION OF ANTIGRAVITY MUSCLES TENSION and TACTILE REACTIVITY - superficial sensation, delicate touch - Avoiding r=-0.3 p<0.05. With an increase of DISTRIBUTION OF ANTIGRAVITY MUSCLES TENSION, TACTILE REACTIVITY - superficial sensation, delicate touch - Avoiding is decreasing
Statistically significant relations are observed between DISTRIBUTION OF ANTIGRAVITY MUSCLES TENSION and TACTILE REACTIVITY - deep sensibility, touch with pressure, and proprioception - Avoiding r=0.16 p<0.05. With an increase of DISTRIBUTION OF ANTIGRAVITY MUSCLES TENSION, TACTILE REACTIVITY - deep sensibility, touch with pressure, and proprioception - Avoiding is increasing
Statistically significant relations are observed between DISTRIBUTION OF ANTIGRAVITY MUSCLES TENSION and VESTIBULAR REACTIVITY - rotary motion - Sensitivity r=0.23 p<0.05. With an increase of DISTRIBUTION OF ANTIGRAVITY MUSCLES TENSION, VESTIBULAR REACTIVITY - rotary motion - Sensitivity is increasing
Statistically significant relations are observed between DISTRIBUTION OF ANTIGRAVITY MUSCLES TENSION and VESTIBULAR REACTIVITY- rotary motion - Avoiding r=0.25 p<0.05. With an increase of DISTRIBUTION OF ANTIGRAVITY MUSCLES TENSION, VESTIBULAR REACTIVITY - rotary motion - Avoiding is increasing
Statistically significant relations are observed between DISTRIBUTION OF ANTIGRAVITY MUSCLES TENSION and VESTIBULAR REACTIVITY - linear motion, variations of the body position in the space - Sensitivity r=0.13 p<0.05. With an increase of DISTRIBUTION OF ANTIGRAVITY MUSCLES TENSION, VESTIBULAR REACTIVITY - linear motion, variations of the body position in the space - Sensitivity is increasing
Statistically significant relations are observed between DISTRIBUTION OF ANTIGRAVITY MUSCLES TENSION and VESTIBULAR REACTIVITY - linear motion, variations of the body position in the space - Avoiding r=0.14 p<0.05. With an increase of DISTRIBUTION OF ANTIGRAVITY MUSCLES TENSION, VESTIBULAR REACTIVITY - linear motion, variations of the body position in the space - Avoiding is increasing
Statistically significant relations are observed between DISTRIBUTION OF ANTIGRAVITY MUSCLES TENSION and OLFACTORY REACTIVITY - Sensitivity r=-0.14 p<0.05. With an increase of DISTRIBUTION OF ANTIGRAVITY MUSCLES TENSION, OLFACTORY REACTIVITY - Sensitivity is decreasing
Statistically significant relations are observed between DISTRIBUTION OF ANTIGRAVITY MUSCLES TENSION and OLFACTORY REACTIVITY - Avoiding r=-0.15 p<0.05. With an increase of DISTRIBUTION OF ANTIGRAVITY MUSCLES TENSION, OLFACTORY REACTIVITY - Avoiding is decreasing

No other statistically significant relations between the indicators are observed.

### Correlations of test indicators vs. questionnaire indicators

Statistically significant relations are observed between TACTILE HYPERSENSITIVITY and TACTILE REACTIVITY - superficial sensation, delicate touch - Sensitivity r=0.41 p<0.05. With an increase of TACTILE HYPERSENSITIVITY, TACTILE REACTIVITY - superficial sensation, delicate touch - Sensitivity is increasing
Statistically significant relations are observed between TACTILE HYPERSENSITIVITY and TACTILE REACTIVITY - superficial sensation, delicate touch - Avoiding r=0.37 p<0.05. With an increase of TACTILE HYPERSENSITIVITY, TACTILE REACTIVITY - superficial sensation, delicate touch - Avoiding is increasing
Statistically significant relations are observed between TACTILE HYPERSENSITIVITY and TACTILE REACTIVITY - deep sensibility, touch with pressure, and proprioception - Sensitivity r=-0.23 p<0.05. With an increase of TACTILE HYPERSENSITIVITY, TACTILE REACTIVITY - deep sensibility, touch with pressure, and proprioception - Sensitivity is decreasing
Statistically significant relations are observed between TACTILE HYPERSENSITIVITY and TACTILE REACTIVITY - deep sensibility, touch with pressure, and proprioception - Avoiding r=-0.2 p<0.05. With an increase of TACTILE HYPERSENSITIVITY, TACTILE REACTIVITY - deep sensibility, touch with pressure, and proprioception - Avoiding is decreasing
Statistically significant relations are observed between TACTILE HYPERSENSITIVITY and VESTIBULAR REACTIVITY - rotary motion - Sensitivity r=-0.17 p<0.05. With an increase of TACTILE HYPERSENSITIVITY, VESTIBULAR REACTIVITY - rotary motion - Sensitivity is decreasing
Statistically significant relations are observed between TACTILE HYPERSENSITIVITY and VESTIBULAR REACTIVITY - rotary motion - Avoiding r=-0.18 p<0.05. With an increase of TACTILE HYPERSENSITIVITY, VESTIBULAR REACTIVITY - rotary motion - Avoiding is decreasing
Statistically significant relations are observed between TACTILE HYPERSENSITIVITY and VESTIBULAR REACTIVITY - linear motion, variations of the body position in the space - Sensitivity r=-0.17 p<0.05. With an increase of TACTILE HYPERSENSITIVITY, VESTIBULAR REACTIVITY - linear motion, variations of the body position in the space - Sensitivity is decreasing
Statistically significant relations are observed between TACTILE HYPERSENSITIVITY and VESTIBULAR REACTIVITY - linear motion, variations of the body position in the space - Avoiding r=-0.15 p<0.05. With an increase of TACTILE HYPERSENSITIVITY, VESTIBULAR REACTIVITY - linear motion, variations of the body position in the space - Avoiding is decreasing
Statistically significant relations are observed between TACTILE HYPERSENSITIVITY and AUDITORY REACTIVITY - sounds produced with instruments - Sensitivity r=0.17 p<0.05. With an increase of TACTILE HYPERSENSITIVITY, AUDITORY REACTIVITY - sounds produced with instruments - Sensitivity is increasing
Statistically significant relations are observed between TACTILE HYPERSENSITIVITY and AUDITORY REACTIVITY - sounds produced with instruments - Avoiding r=0.14 p<0.05. With an increase of TACTILE HYPERSENSITIVITY, AUDITORY REACTIVITY - sounds produced with instruments - Avoiding is increasing
Statistically significant relations are observed between TACTILE HYPERSENSITIVITY and AUDITORY REACTIVITY- sounds reproduced - Sensitivity r=0.14 p<0.05. With an increase of TACTILE HYPERSENSITIVITY, AUDITORY REACTIVITY- sounds reproduced - Sensitivity is increasing
Statistically significant relations are observed between TACTILE HYPERSENSITIVITY and AUDITORY REACTIVITY- sounds reproduced - Avoiding r=0.14 p<0.05. With an increase of TACTILE HYPERSENSITIVITY, AUDITORY REACTIVITY- sounds reproduced - Avoiding is increasing
Statistically significant relations are observed between TACTILE HYPERSENSITIVITY and VISUAL REACTIVITY - Sensitivity r=0.21 p<0.05. With an increase of TACTILE HYPERSENSITIVITY, VISUAL REACTIVITY - Sensitivity is increasing
Statistically significant relations are observed between TACTILE HYPERSENSITIVITY and VISUAL REACTIVITY - Avoiding r=0.16 p<0.05. With an increase of TACTILE HYPERSENSITIVITY, increasing is VISUAL REACTIVITY - Avoiding
Statistically significant relations are observed between TACTILE HYPERSENSITIVITY and BALANCE r=-0.29 p<0.05. With an increase of TACTILE HYPERSENSITIVITY, BALANCE is decreasing.
Statistically significant relations are observed between TACTILE HYPERSENSITIVITY and MANUAL PRAXES r=-0.36 p<0.05. With an increase of TACTILE HYPERSENSITIVITY, MANUAL PRAXES is decreasing
Statistically significant relations are observed between TACTILE HYPERSENSITIVITY and POSTURAL PRAXES r=-0.22 p<0.05. With an increase of TACTILE HYPERSENSITIVITY, POSTURAL PRAXES is decreasing
Statistically significant relations are observed between TACTILE HYPERSENSITIVITY and DISTRIBUTION OF ANTIGRAVITY MUSCLES TENSION r=-0.21 p<0.05. With an increase of TACTILE HYPERSENSITIVITY, DISTRIBUTION OF ANTIGRAVITY MUSCLES TENSION is decreasing
Statistically significant relations are observed between VESTIBULAR HYPERSENSITIVITY and BALANCE r=0.15 p<0.05. With an increase of VESTIBULAR HYPERSENSITIVITY, BALANCE is increasing
Statistically significant relations are observed between AUDITORY HYPERSENSITIVITY and TACTILE REACTIVITY - superficial sensation, delicate touch - Sensitivity r=0.19 p<0.05. With an increase of AUDITORY HYPERSENSITIVITY, TACTILE REACTIVITY - superficial sensation, delicate touch - Sensitivity is increasing
Statistically significant relations are observed between AUDITORY HYPERSENSITIVITY and TACTILE REACTIVITY - superficial sensation, delicate touch - Avoiding r=0.14 p<0.05. With an increase of AUDITORY HYPERSENSITIVITY, TACTILE REACTIVITY - superficial sensation, delicate touch - Avoiding is increasing
Statistically significant relations are observed between AUDITORY HYPERSENSITIVITY and TACTILE REACTIVITY - deep sensibility, touch with pressure, and proprioception - Sensitivity r=-0.17 p<0.05. With an increase of AUDITORY HYPERSENSITIVITY, TACTILE REACTIVITY - deep sensibility, touch with pressure, and proprioception - Sensitivity is decreasing
Statistically significant relations are observed between AUDITORY HYPERSENSITIVITY and TACTILE REACTIVITY - deep sensibility, touch with pressure, and proprioception - Avoiding r=-0.15 p<0.05. With an increase of AUDITORY HYPERSENSITIVITY, TACTILE REACTIVITY - deep sensibility, touch with pressure, and proprioception - Avoiding is decreasing
Statistically significant relations are observed between AUDITORY HYPERSENSITIVITY and AUDITORY REACTIVITY - sounds produced with instruments - Sensitivity r=0.25 p<0.05. With an increase of AUDITORY HYPERSENSITIVITY, AUDITORY REACTIVITY - sounds produced with instruments - Sensitivity is increasing
Statistically significant relations are observed between AUDITORY HYPERSENSITIVITY and AUDITORY REACTIVITY - sounds produced with instruments - Avoiding r=0.24 p<0.05. With an increase of AUDITORY HYPERSENSITIVITY, AUDITORY REACTIVITY - sounds produced with instruments - Avoiding is increasing
Statistically significant relations are observed between AUDITORY HYPERSENSITIVITY and AUDITORY REACTIVITY- sounds reproduced - Sensitivity r=0.32 p<0.05. With an increase of AUDITORY HYPERSENSITIVITY, AUDITORY REACTIVITY- sounds reproduced - Sensitivity is increasing
Statistically significant relations are observed between AUDITORY HYPERSENSITIVITY and AUDITORY REACTIVITY- sounds reproduced - Avoiding r=0.27 p<0.05. With an increase of AUDITORY HYPERSENSITIVITY, AUDITORY REACTIVITY- sounds reproduced - Avoiding is increasing
Statistically significant relations are observed between AUDITORY HYPERSENSITIVITY and VISUAL REACTIVITY - Sensitivity r=0.2 p<0.05. With an increase of AUDITORY HYPERSENSITIVITY, VISUAL REACTIVITY - Sensitivity is increasing
Statistically significant relations are observed between AUDITORY HYPERSENSITIVITY and VISUAL REACTIVITY - Avoiding r=0.18 p<0.05. With an increase of AUDITORY HYPERSENSITIVITY, VISUAL REACTIVITY - Avoiding is increasing
Statistically significant relations are observed between AUDITORY HYPERSENSITIVITY and BALANCE r=-0.28 p<0.05. With an increase of AUDITORY HYPERSENSITIVITY, BALANCE is decreasing
Statistically significant relations are observed between AUDITORY HYPERSENSITIVITY and VISUAL-MOTOR COORDINATION r=-0.13 p<0.05. With an increase of AUDITORY HYPERSENSITIVITY, VISUAL-MOTOR COORDINATION is decreasing
Statistically significant relations are observed between OLFACTORY HYPERSENSITIVITY and TACTILE REACTIVITY - superficial sensation, delicate touch - Sensitivity r=0.16 p<0.05. With an increase of OLFACTORY HYPERSENSITIVITY, TACTILE REACTIVITY - superficial sensation, delicate touch - Sensitivity is increasing
Statistically significant relations are observed between OLFACTORY HYPERSENSITIVITY and TACTILE REACTIVITY - superficial sensation, delicate touch - Avoiding r=0.14 p<0.05. With an increase of OLFACTORY HYPERSENSITIVITY, TACTILE REACTIVITY - superficial sensation, delicate touch - Avoiding is increasing
Statistically significant relations are observed between OLFACTORY HYPERSENSITIVITY and TACTILE REACTIVITY - deep sensibility, touch with pressure, and proprioception - Sensitivity r=-0.23 p<0.05. With an increase of OLFACTORY HYPERSENSITIVITY, TACTILE REACTIVITY - deep sensibility, touch with pressure, and proprioception - Sensitivity is decreasing
Statistically significant relations are observed between OLFACTORY HYPERSENSITIVITY and TACTILE REACTIVITY - deep sensibility, touch with pressure, and proprioception - Avoiding r=-0.18 p<0.05. With an increase of OLFACTORY HYPERSENSITIVITY, TACTILE REACTIVITY - deep sensibility, touch with pressure, and proprioception - Avoiding is decreasing
Statistically significant relations are observed between OLFACTORY HYPERSENSITIVITY and VESTIBULAR REACTIVITY- rotary motion - Sensitivity r=-0.2 p<0.05. With an increase of OLFACTORY HYPERSENSITIVITY, VESTIBULAR REACTIVITY - rotary motion - Sensitivity is decreasing
Statistically significant relations are observed between OLFACTORY HYPERSENSITIVITY and VESTIBULAR REACTIVITY- rotary motion - Avoiding r=-0.19 p<0.05. With an increase of OLFACTORY HYPERSENSITIVITY, VESTIBULAR REACTIVITY - rotary motion - Avoiding is decreasing
Statistically significant relations are observed between OLFACTORY HYPERSENSITIVITY and VESTIBULAR REACTIVITY - linear motion, variations of the body position in the space - Sensitivity r=-0.16 p<0.05. With an increase of OLFACTORY HYPERSENSITIVITY, VESTIBULAR REACTIVITY - linear motion, variations of the body position in the space - Sensitivity is decreasing
Statistically significant relations are observed between OLFACTORY HYPERSENSITIVITY and VESTIBULAR REACTIVITY - linear motion, variations of the body position in the space - Avoiding r=-0.16 p<0.05. With an increase of OLFACTORY HYPERSENSITIVITY, VESTIBULAR REACTIVITY - linear motion, variations of the body position in the space - Avoiding is decreasing
Statistically significant relations are observed between OLFACTORY HYPERSENSITIVITY and BALANCE r=-0.15 p<0.05. With an increase of OLFACTORY HYPERSENSITIVITY, BALANCE is decreasing
Statistically significant relations are observed between OLFACTORY HYPERSENSITIVITY and PRAXES r=0.24 p<0.05. With an increase of OLFACTORY HYPERSENSITIVITY, PRAXES is increasing
Statistically significant relations are observed between OLFACTORY HYPERSENSITIVITY and DISTRIBUTION OF ANTIGRAVITY MUSCLES TENSION r=-0.14 p<0.05. With an increase of OLFACTORY HYPERSENSITIVITY, DISTRIBUTION OF ANTIGRAVITY MUSCLES TENSION is decreasing
Statistically significant relations are observed between VISUAL HYPERSENSITIVITY and TACTILE REACTIVITY - superficial sensation, delicate touch - Sensitivity r=0.34 p<0.05. With an increase of VISUAL HYPERSENSITIVITY, TACTILE REACTIVITY - superficial sensation, delicate touch - Sensitivity is increasing
Statistically significant relations are observed between VISUAL HYPERSENSITIVITY and TACTILE REACTIVITY - superficial sensation, delicate touch - Avoiding r=0.29 p<0.05. With an increase of VISUAL HYPERSENSITIVITY, TACTILE REACTIVITY - superficial sensation, delicate touch - Avoiding is increasing
Statistically significant relations are observed between VISUAL HYPERSENSITIVITY and TACTILE REACTIVITY - deep sensibility, touch with pressure, and proprioception - Sensitivity r=-0.19 p<0.05. With an increase of VISUAL HYPERSENSITIVITY, TACTILE REACTIVITY - deep sensibility, touch with pressure, and proprioception - Sensitivity is decreasing
Statistically significant relations are observed between VISUAL HYPERSENSITIVITY and TACTILE REACTIVITY - deep sensibility, touch with pressure, and proprioception - Avoiding r=-0.19 p<0.05. With an increase of VISUAL HYPERSENSITIVITY, TACTILE REACTIVITY - deep sensibility, touch with pressure, and proprioception - Avoiding is decreasing
Statistically significant relations are observed between VISUAL HYPERSENSITIVITY and VESTIBULAR REACTIVITY- rotary motion - Sensitivity r=-0.14 p<0.05. With an increase of VISUAL HYPERSENSITIVITY, VESTIBULAR REACTIVITY - rotary motion - Sensitivity is decreasing
Statistically significant relations are observed between VISUAL HYPERSENSITIVITY and VESTIBULAR REACTIVITY- rotary motion - Avoiding r=-0.14 p<0.05. With an increase of VISUAL HYPERSENSITIVITY, VESTIBULAR REACTIVITY - rotary motion - Avoiding is decreasing
Statistically significant relations are observed between VISUAL HYPERSENSITIVITY and AUDITORY REACTIVITY- sounds reproduced - Sensitivity r=0.15 p<0.05. With an increase of VISUAL HYPERSENSITIVITY, AUDITORY REACTIVITY- sounds reproduced - Sensitivity is increasing
Statistically significant relations are observed between VISUAL HYPERSENSITIVITY and VISUAL REACTIVITY - Sensitivity r=0.16 p<0.05. With an increase of VISUAL HYPERSENSITIVITY, VISUAL REACTIVITY - Sensitivity is increasing
Statistically significant relations are observed between VISUAL HYPERSENSITIVITY and BALANCE r=-0.17 p<0.05. With an increase of VISUAL HYPERSENSITIVITY, BALANCE is decreasing
Statistically significant relations are observed between VISUAL HYPERSENSITIVITY and DISTRIBUTION OF ANTIGRAVITY MUSCLES TENSION r=-0.13 p<0.05. With an increase of VISUAL HYPERSENSITIVITY, DISTRIBUTION OF ANTIGRAVITY MUSCLES TENSION is decreasing
Statistically significant relations are observed between DECREASED MUSCLE TONE and TACTILE REACTIVITY - superficial sensation, delicate touch - Sensitivity r=0.2 p<0.05. With an increase of DECREASED MUSCLE TONE, TACTILE REACTIVITY - superficial sensation, delicate touch - Sensitivity is increasing
Statistically significant relations are observed between DECREASED MUSCLE TONE and TACTILE REACTIVITY - superficial sensation, delicate touch - Avoiding r=0.16 p<0.05. With an increase of DECREASED MUSCLE TONE, TACTILE REACTIVITY - superficial sensation, delicate touch - Avoiding is increasing
Statistically significant relations are observed between DECREASED MUSCLE TONE and AUDITORY REACTIVITY - sounds produced with instruments - Sensitivity r=0.13 p<0.05. With an increase of DECREASED MUSCLE TONE, AUDITORY REACTIVITY - sounds produced with instruments - Sensitivity is increasing
Statistically significant relations are observed between DECREASED MUSCLE TONE and BALANCE r=-0.17 p<0.05. With an increase of DECREASED MUSCLE TONE, BALANCE is decreasing
Statistically significant relations are observed between DECREASED MUSCLE TONE and MANUAL PRAXES r=-0.25 p<0.05. With an increase of DECREASED MUSCLE TONE, MANUAL PRAXES is decreasing
Statistically significant relations are observed between DECREASED MUSCLE TONE and POSTURAL PRAXES r=-0.24 p<0.05. With an increase of DECREASED MUSCLE TONE, POSTURAL PRAXES is decreasing
Statistically significant relations are observed between DECREASED MUSCLE TONE and REFLEX INTEGRATION r=-0.18 p<0.05. With an increase of DECREASED MUSCLE TONE, REFLEX INTEGRATION is decreasing
Statistically significant relations are observed between DECREASED MUSCLE TONE and DISTRIBUTION OF ANTIGRAVITY MUSCLES TENSION r=-0.23 p<0.05. With an increase of DECREASED MUSCLE TONE, DISTRIBUTION OF ANTIGRAVITY MUSCLES TENSION is decreasing
Statistically significant relations are observed between AUDITORY HYPOSENSITIVITY and TACTILE REACTIVITY - superficial sensation, delicate touch - Sensitivity r=-0.24 p<0.05. With an increase of AUDITORY HYPOSENSITIVITY, TACTILE REACTIVITY - superficial sensation, delicate touch - Sensitivity is decreasing
Statistically significant relations are observed between AUDITORY HYPOSENSITIVITY and TACTILE REACTIVITY - superficial sensation, delicate touch - Avoiding r=-0.2 p<0.05. With an increase of AUDITORY HYPOSENSITIVITY, TACTILE REACTIVITY - superficial sensation, delicate touch - Avoiding is decreasing
Statistically significant relations are observed between AUDITORY HYPOSENSITIVITY and TACTILE REACTIVITY - deep sensibility, touch with pressure, and proprioception - Sensitivity r=0.2 p<0.05. With an increase of AUDITORY HYPOSENSITIVITY, TACTILE REACTIVITY - deep sensibility, touch with pressure, and proprioception - Sensitivity is increasing
Statistically significant relations are observed between AUDITORY HYPOSENSITIVITY and TACTILE REACTIVITY - deep sensibility, touch with pressure, and proprioception - Avoiding r=0.2 p<0.05. With an increase of AUDITORY HYPOSENSITIVITY, TACTILE REACTIVITY - deep sensibility, touch with pressure, and proprioception - Avoiding is increasing
Statistically significant relations are observed between AUDITORY HYPOSENSITIVITY and AUDITORY REACTIVITY - sounds produced with instruments - Sensitivity r=-0.3 p<0.05. With an increase of AUDITORY HYPOSENSITIVITY, AUDITORY REACTIVITY - sounds produced with instruments - Sensitivity is decreasing
Statistically significant relations are observed between AUDITORY HYPOSENSITIVITY and AUDITORY REACTIVITY - sounds produced with instruments - Avoiding r=-0.23 p<0.05. With an increase of AUDITORY HYPOSENSITIVITY, AUDITORY REACTIVITY - sounds produced with instruments - Avoiding is decreasing
Statistically significant relations are observed between AUDITORY HYPOSENSITIVITY and AUDITORY REACTIVITY- sounds reproduced - Sensitivity r=-0.34 p<0.05. With an increase of AUDITORY HYPOSENSITIVITY, AUDITORY REACTIVITY- sounds reproduced - Sensitivity is decreasing
Statistically significant relations are observed between AUDITORY HYPOSENSITIVITY and AUDITORY REACTIVITY- sounds reproduced - Avoiding r=-0.27 p<0.05. With an increase of AUDITORY HYPOSENSITIVITY, AUDITORY REACTIVITY- sounds reproduced - Avoiding is decreasing
Statistically significant relations are observed between AUDITORY HYPOSENSITIVITY and OLFACTORY REACTIVITY - Sensitivity r=-0.16 p<0.05. With an increase of AUDITORY HYPOSENSITIVITY, OLFACTORY REACTIVITY - Sensitivity is decreasing
Statistically significant relations are observed between DEEP SENSIBILITY HYPOSENSITIVITY, proprioception and OCULOMOTOR CONTROL r=-0.17 p<0.05. With an increase of DEEP SENSIBILITY HYPOSENSITIVITY, proprioception, OCULOMOTOR CONTROL is decreasing
Statistically significant relations are observed between DEEP SENSIBILITY HYPOSENSITIVITY, proprioception and PRAXES r=-0.18 p<0.05. With an increase of DEEP SENSIBILITY HYPOSENSITIVITY, proprioception, PRAXES is decreasing
Statistically significant relations are observed between DEEP SENSIBILITY HYPOSENSITIVITY, proprioception and REFLEX INTEGRATION r=-0.26 p<0.05. With an increase of DEEP SENSIBILITY HYPOSENSITIVITY, proprioception, REFLEX INTEGRATION is decreasing
Statistically significant relations are observed between OLFACTORY HYPOSENSITIVITY and TACTILE REACTIVITY - deep sensibility, touch with pressure, and proprioception - Sensitivity r=0.2 p<0.05. With an increase of OLFACTORY HYPOSENSITIVITY, TACTILE REACTIVITY - deep sensibility, touch with pressure, and proprioception - Sensitivity is increasing
Statistically significant relations are observed between OLFACTORY HYPOSENSITIVITY and TACTILE REACTIVITY - deep sensibility, touch with pressure, and proprioception - Avoiding r=0.15 p<0.05. With an increase of OLFACTORY HYPOSENSITIVITY, TACTILE REACTIVITY - deep sensibility, touch with pressure, and proprioception - Avoiding is increasing
Statistically significant relations are observed between OLFACTORY HYPOSENSITIVITY and VESTIBULAR REACTIVITY - rotary motion - Sensitivity r=0.15 p<0.05. With an increase of OLFACTORY HYPOSENSITIVITY, VESTIBULAR REACTIVITY - rotary motion - Sensitivity is increasing
Statistically significant relations are observed between OLFACTORY HYPOSENSITIVITY and VESTIBULAR REACTIVITY - rotary motion - Avoiding r=0.13 p<0.05. With an increase of OLFACTORY HYPOSENSITIVITY, VESTIBULAR REACTIVITY - rotary motion - Avoiding is increasing
Statistically significant relations are observed between OLFACTORY HYPOSENSITIVITY and VESTIBULAR REACTIVITY - linear motion, variations of the body position in the space - Sensitivity r=0.16 p<0.05. With an increase of OLFACTORY HYPOSENSITIVITY, VESTIBULAR REACTIVITY - linear motion, variations of the body position in the space - Sensitivity is increasing
Statistically significant relations are observed between OLFACTORY HYPOSENSITIVITY and VESTIBULAR REACTIVITY - linear motion, variations of the body position in the space - Avoiding r=0.16 p<0.05. With an increase of OLFACTORY HYPOSENSITIVITY, VESTIBULAR REACTIVITY - linear motion, variations of the body position in the space - Avoiding is increasing
Statistically significant relations are observed between OLFACTORY HYPOSENSITIVITY and PRAXES r=-0.23 p<0.05. With an increase of OLFACTORY HYPOSENSITIVITY, PRAXES is decreasing
Statistically significant relations are observed between VISUAL HYPOSENSITIVITY and VISUAL REACTIVITY - Avoiding r=-0.15 p<0.05. With an increase of VISUAL HYPOSENSITIVITY, VISUAL REACTIVITY - Avoiding is decreasing
Statistically significant relations are observed between VISUAL HYPOSENSITIVITY and REFLEX INTEGRATION r=-0.2 p<0.05. With an increase of VISUAL HYPOSENSITIVITY, REFLEX INTEGRATION is decreasing
Statistically significant relations are observed between VISUAL-MOTOR COORDINATION DISORDERS and TACTILE REACTIVITY - superficial sensation, delicate touch - Sensitivity r=0.27 p<0.05. With an increase of VISUAL-MOTOR COORDINATION DISORDERS, TACTILE REACTIVITY - superficial sensation, delicate touch - Sensitivity is increasing
Statistically significant relations are observed between VISUAL-MOTOR COORDINATION DISORDERS and TACTILE REACTIVITY - superficial sensation, delicate touch - Avoiding r=0.27 p<0.05. With an increase of VISUAL-MOTOR COORDINATION DISORDERS, TACTILE REACTIVITY - superficial sensation, delicate touch - Avoiding is increasing
Statistically significant relations are observed between VISUAL-MOTOR COORDINATION DISORDERS and TACTILE REACTIVITY - deep sensibility, touch with pressure, and proprioception - Sensitivity r=-0.16 p<0.05. With an increase of VISUAL-MOTOR COORDINATION DISORDERS, TACTILE REACTIVITY - deep sensibility, touch with pressure, and proprioception - Sensitivity is decreasing
Statistically significant relations are observed between VISUAL-MOTOR COORDINATION DISORDERS and AUDITORY REACTIVITY - sounds produced with instruments - Sensitivity r=0.16 p<0.05. With an increase of VISUAL-MOTOR COORDINATION DISORDERS, AUDITORY REACTIVITY - sounds produced with instruments - Sensitivity is increasing
Statistically significant relations are observed between VISUAL-MOTOR COORDINATION DISORDERS and BALANCE r=-0.4 p<0.05. With an increase of VISUAL-MOTOR COORDINATION DISORDERS, BALANCE is decreasing
Statistically significant relations are observed between VISUAL-MOTOR COORDINATION DISORDERS and PRAXES r=-0.37 p<0.05. With an increase of VISUAL-MOTOR COORDINATION DISORDERS, PRAXES is decreasing
Statistically significant relations are observed between VISUAL-MOTOR COORDINATION DISORDERS and MANUAL PRAXES r=-0.47 p<0.05. With an increase of VISUAL-MOTOR COORDINATION DISORDERS, MANUAL PRAXES is decreasing
Statistically significant relations are observed between VISUAL-MOTOR COORDINATION DISORDERS and POSTURAL PRAXES r=-0.37 p<0.05. With an increase of VISUAL-MOTOR COORDINATION DISORDERS, POSTURAL PRAXES is decreasing
Statistically significant relations are observed between VISUAL-MOTOR COORDINATION DISORDERS and REFLEX INTEGRATION r=-0.2 p<0.05. With an increase of VISUAL-MOTOR COORDINATION DISORDERS, REFLEX INTEGRATION is decreasing
Statistically significant relations are observed between VISUAL-MOTOR COORDINATION DISORDERS and VISUAL-MOTOR COORDINATION r=-0.38 p<0.05. With an increase of VISUAL-MOTOR COORDINATION DISORDERS, VISUAL-MOTOR COORDINATION is decreasing
Statistically significant relations are observed between VISUAL-MOTOR COORDINATION DISORDERS and DISTRIBUTION OF ANTIGRAVITY MUSCLES TENSION r=-0.22 p<0.05. With an increase of VISUAL-MOTOR COORDINATION DISORDERS, DISTRIBUTION OF ANTIGRAVITY MUSCLES TENSION is decreasing
Statistically significant relations are observed between BALANCE DISORDERS and TACTILE REACTIVITY - superficial sensation, delicate touch - Sensitivity r=0.22 p<0.05. With an increase of BALANCE DISORDERS, TACTILE REACTIVITY - superficial sensation, delicate touch - Sensitivity is increasing
Statistically significant relations are observed between BALANCE DISORDERS and TACTILE REACTIVITY - superficial sensation, delicate touch - Avoiding r=0.21 p<0.05. With an increase of BALANCE DISORDERS, TACTILE REACTIVITY - superficial sensation, delicate touch - Avoiding is increasing
Statistically significant relations are observed between BALANCE DISORDERS and AUDITORY REACTIVITY - sounds produced with instruments - Sensitivity r=0.18 p<0.05. With an increase of BALANCE DISORDERS, AUDITORY REACTIVITY - sounds produced with instruments - Sensitivity is increasing
Statistically significant relations are observed between BALANCE DISORDERS and AUDITORY REACTIVITY - sounds produced with instruments - Avoiding r=0.16 p<0.05. With an increase of BALANCE DISORDERS, AUDITORY REACTIVITY - sounds produced with instruments - Avoiding is increasing
Statistically significant relations are observed between BALANCE DISORDERS and BALANCE r=-0.21 p<0.05. With an increase of BALANCE DISORDERS, BALANCE is decreasing

No other statistically significant relations between indicators are observed.

### Summary of the analyses

The aim of the analyses was to find ranges of variables and important factors that could indicate the lack of or the presence of disorders.

In the analysis, children were compared in the age ranges, in groups with disorders and without disorders in terms of analyzed variables.

The possibility of assigning children to groups with disorders based on increased only one variable of the questionnaire scale imposes another approach than used so far by SI therapists for prognosing the disorder based on questionnaire scales.

Taking into consideration that the therapists were assigning children to groups with disorders only based on tests results following an adopted scheme (for example a result above some value was interpreted as the presence of disorders), one should clearly define the range in which therapists diagnose a given disorder.

The presently carried out analyses give answers to the questions:
- In terms of which variables the children with disorders differed from those without disorders - in other words, which disorders occur most frequently in the population?
- Does the age affect the range of test scales?
- What were the characteristics of the tested group and the control group in terms of variables of the reconnaissance?
- What are the relations between individual variables both in the group of children with disorders and without disorders?
- What are the results of particular disorders in the population?

### II Normalization of the questionnaire

The following analysis related to creating ranges for questionnaire and test scales.

The ranges included: norms, risks, and disorders.

### Introduction

Therapists qualified users to groups of children with disorders and without disorders based on the Test of Sensory Processing Disorders and therapeutic observation.

Because of the lack of information about the nature of the disorders, the group of patients with disorders included patients who had some of the indicators normal.

On the contrary, the group of the users without disorders could include some users who had some deviations from the norm, but after the reconnaissance and observation, the therapist could state no such disorders.

Because of the nature of grouping the users by therapists without information about the types and degrees of disorders, it is problematic to create, on this basis, the ranges of particular indicators defining the level of the norm and disorders by comparing the groups of the users with disorders and without disorders.

Taking into consideration the above, it was decided to concentrate on the group without disorders when creating the normalization/reference scales for particular types of disorders and treat it as the starting point when creating the normalization of the test.

Finally, it was adopted that the norm is located at a distance of one standard deviation from the average, the range of risk at a distance from the first to the second standard deviation from the average, and the range below or above the second standard deviation up to extreme results has been marked as a disorder.

### Test of Sensory Modulation Disorders

For the scales of the Test of Sensory Modulation Disorders it has been adopted that the norm is located at a distance of one standard deviation from the average, the range of risk at a distance from the first to the second standard deviation from the average, and the range below or above the second standard deviation up to extreme results has been marked as a disorder.

### Example for TACTILE REACTIVITY - superficial sensation, delicate touch - Sensitivity

Disorder from 0 to 21; from 29 to 42;
Risk from 21 to 23; from 27 to 29;
Norm from 23 to 27;

| Indicator | Min. result | M-2sd | M-1sd | M | M+1sd | M+2sd | Max. result |
|---|---|---|---|---|---|---|---|
| TACTILE REACTIVITY - superficial sensation, delicate touch - Sensitivity | 0 | 21.0 | 23.1 | 25.2 | 27.2 | 29.3 | 42 |
| TACTILE REACTIVITY - superficial sensation, delicate touch - Avoiding | 0 | 21.0 | 23.1 | 25.3 | 27.4 | 29.6 | 42 |
| TACTILE REACTIVITY - deep sensibility, touch with pressure, and proprioception - Sensitivity | 0 | 18.8 | 21.2 | 23.5 | 25.9 | 28.2 | 42 |
| TACTILE REACTIVITY - deep sensibility, touch with pressure, and proprioception - Avoiding | 0 | 18.1 | 20.5 | 23.0 | 25.5 | 27.9 | 42 |
| VESTIBULAR REACTIVITY - rotary motion - Sensitivity | 0 | 16.9 | 20.4 | 23.8 | 27.3 | 30.8 | 42 |
| VESTIBULAR REACTIVITY - rotary motion - Avoiding | 0 | 16.1 | 19.8 | 23.6 | 27.3 | 31.1 | 42 |
| VESTIBULAR REACTIVITY - linear motion, variations of the body position in the space - Sensitivity | 0 | 20.5 | 22.3 | 24.1 | 25.8 | 27.6 | 42 |
| VESTIBULAR REACTIVITY - linear motion, variations of the body position in the space - Avoiding | 0 | 18.6 | 21.0 | 23.5 | 25.9 | 28.4 | 42 |
| AUDITORY REACTIVITY - sounds produced with instruments - Sensitivity | 0 | 19.2 | 21.7 | 24.3 | 26.8 | 29.4 | 42 |
| AUDITORY REACTIVITY - sounds produced with instruments - Avoiding | 0 | 17.9 | 20.8 | 23.7 | 26.6 | 29.5 | 42 |
| AUDITORY REACTIVITY- sounds reproduced - Sensitivity | 0 | 19.9 | 22.0 | 24.2 | 26.3 | 28.5 | 42 |
| AUDITORY REACTIVITY- sounds reproduced - Avoiding | 0 | 21.4 | 23.0 | 24.6 | 26.1 | 27.7 | 42 |
| VISUAL REACTIVITY - Sensitivity | 0 | 20.3 | 22.3 | 24.3 | 26.3 | 28.3 | 42 |
| VISUAL REACTIVITY - Avoiding | 0 | 18.9 | 21.7 | 24.4 | 27.2 | 30.0 | 42 |
| OLFACTORY REACTIVITY - Sensitivity | 0 | 21.3 | 23.1 | 24.9 | 26.7 | 28.4 | 42 |
| OLFACTORY REACTIVITY - Avoiding | 0 | 20.2 | 22.4 | 24.7 | 26.9 | 29.2 | 42 |

### Test of Motor Disorders with Sensory Basis

For scales of praxes it has been noticed that the way therapists mark answers in the group without disorders would suggest disorders, in this scale, for most of healthy people. This fact raises objections as far as the interpretation of analyses is concerned. A conclusion may be drawn that some therapists could understand a higher score as a pathology whereas others as the norm. A similar situation may be observed for the scale Visual-motor coordination. Hence, a bimodal graph occurs frequently in the results of persons without disorders. As has been noticed, in the Test Of Motor Disorders with Sensory Basis there is no instruction that would be the reference for therapists stating if a higher score denotes the norm or a pathology. When doing this part of the test, the therapists followed their knowledge acquired during courses.

One should emphasize that the indicated ranges must be treated approximately, and the final definition of the ranges should be given and adopted by the professionals.

For scales of the Test of Motor Disorders with Sensory Basis it has been adopted that the results in the range from the lowest to the average minus two standard deviations define the presence of disorders, the results in the range from the average minus two standard deviations to the average minus one standard deviation define the group of risk, and the results of the average minus one standard deviation to maximum score define the norm.

### Example for OCULOMOTOR CONTROL

Disorder from 0 to 2.9;
Risk from 2.9 to 5.9;
Norm from 5.9 to 12;

| Indicator | Min. result | M-2sd | M-1sd | M | M+1sd | Max. result |
|---|---|---|---|---|---|---|
| OCULOMOTOR CONTROL | 0 | 2.9 | 5.9 | 8.8 | 11.8 | 12 |
| BALANCE | 0 | 3.7 | 6.0 | 8.3 | 10.6 | 12 |
| PRAXES ORALNE | 0 | 5.9 | 7.8 | 9.7 | 11.6 | 12 |
| MANUAL PRAXES | 0 | 5.9 | 7.9 | 9.9 | 11.9 | 12 |
| POSTURAL PRAXES | 0 | 5.9 | 7.9 | 9.8 | 11.8 | 12 |
| PRAXES | 0 | 0.0 | 1.7 | 5.2 | 8.8 | 12 |
| REFLEX INTEGRATION | 0 | 3.3 | 5.8 | 8.3 | 10.8 | 12 |
| VISUAL-MOTOR COORDINATION | 0 | 4.0 | 6.6 | 9.2 | 11.8 | 12 |
| DISTRIBUTION OF ANTIGRAVITY MUSCLES TENSION | 0 | 6.1 | 8.1 | 10.1 | 12 | 12 |

### Questionnaire of sensory development

The process of creating the classification of the questionnaire questions based on disorders was as follows.

In order to unify the questionnaire indicators, the questions that were included in indicators, in which the names of the classifications of questions were ended as: "the more points for NO", were recoded reversely (the scale has been reversed). Next, indicators were calculated that were included in the names of the classifications of questions (table) using the arithmetic average.

In the next step, also using the arithmetic average, 13 final indicators have been created:

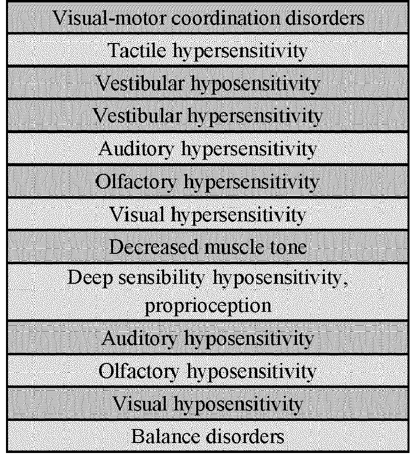

| No | Names of variabl es | Names from the question classification | Recoded variables | Created indicators |
|---|---|---|---|---|
| 2 | a2 | Tactile hypersensitivity of superficial sensation - the more points for NO | ai2 | Tactile hypersensitivity |
| 3 | a3 | Tactile hypersensitivity - points for YES | | |
| 4 | a4 | Hyposensitivity/vestibular seeking - the more points for NO | ai4 | Vestibular hyposensitivity |
| 5 | a5 | Hyposensitivity/vestibular seeking - the more points for YES | | |
| 14 | a14 | Hyposensitivity/vestibular seeking - the more points for NO | ai14 | Vestibular hypersensitivity |
| 15 | a15 | Hyposensitivity/vestibular seeking - points for YES | | |
| 6 | a6 | Auditory hypersensitivity - the more points for NO | ai6 | Auditory hypersensitivity |
| 7 | a7 | Auditory hypersensitivity- the more points for YES | | |
| 8 | a8 | Olfactory hypersensitivity - the more points for NO | ai8 | Olfactory hypersensitivity |
| 9 | a9 | Olfactory hypersensitivity - points for YES | | |
| 10 | a10 | Visual hypersensitivity - the more points for NO | ai10 | Visual hypersensitivity |
| 11 | a11 | Visual hypersensitivity - points for YES | | |
| 12 | a12 | Decreased muscle tone - the more points for YES | | Decreased muscle tone |
| 16 | a16 | Auditory hyposensitivity - the more points for NO | ai16 | Auditory hyposensitivity |
| 17 | a17 | Hyposensitivity / auditory seeking - the more points for YES | | |
| 13 | a13 | Hyposensitivity/deep sensibility seeking and proprioception - points for YES | | Deep sensibility hyposensitivity, proprioception |
| 18 | a18 | Olfactory hyposensitivity - points for YES | | Olfactory hyposensitivity |
| 19 | a19 | Visual hyposensitivity - the more points for NO | ai19 | Visual hyposensitivity |
| 20 | a20 | Visual hyposensitivity - points for YES | | |
| 1 | a1 | Visual-motor coordination disorders - the more points for NO | ai1 | Visual-motor coordination disorders |
| 21 | a21 | Disorders of motor planning motorycznego - the more points for NO | ai21 | |
| 22 | a22 | Visual-motor coordination disorders - the more points for YES | | |
| 23 | a23 | Balance disorders - the more points for NO | ai23 | Balance disorders |
| 24 | a24 | Balance disorders - the more points for YES | | |

| | | | | |
|---|---|---|---|---|
| a- denotes the variable name in the database ai- denotes the variable name in the database, that has been recoded | | | | |

For the questionnaire scales, it has been adopted that the norm is located at a distance of one standard deviation from the average, the range of risk is located at a distance w from the first standard deviation to the second standard deviation from the average, and the range below or above the second standard deviation up to extreme results has been marked as a disorder.

### Example for Tactile hypersensitivity

Disorder from 0 to 2; from 5 to 10;
Risk from 2 to 3; from 4 to 5;
Norm from 3 to 5;

| Indicator | Min. result | M-2sd | M-1sd | M | M+1sd | M+2sd | Max. result |
|---|---|---|---|---|---|---|---|
| Tactile hypersensitivit y | 0 | 2 | 3 | 3 | 4 | 5 | 10 |
| Vestibular hyposensitivity | 0 | 4 | 5 | 5 | 5 | 6 | 10 |
| Vestibular hypersensitivit y | 0 | 4 | 5 | 5 | 5 | 6 | 10 |
| Auditory hypersensitivit y | 0 | 1 | 3 | 4 | 5 | 6 | 10 |
| Olfactory hypersensitivit y | 0 | 0 | 2 | 4 | 6 | 8 | 10 |
| Visual hypersensitivit y | 0 | 2 | 3 | 4 | 5 | 6 | 10 |
| Decreased muscle tone | 0 | 0 | 0 | 2 | 4 | 6 | 10 |
| Auditory hyposensitivity | 0 | 4 | 5 | 6 | 7 | 8 | 10 |
| Deep sensibility hyposensitivity, proprioception | 0 | 2 | 4 | 6 | 8 | 10 | 10 |
| Olfactory hyposensitivity | 0 | 0 | 2 | 5 | 8 | 10 | 10 |
| Visual hyposensitivity | 0 | 2 | 3 | 5 | 6 | 8 | 10 |
| Visual-motor coordination disorders | 0 | 0 | 1 | 2 | 4 | 5 | 10 |
| balance disorders | 0 | 0 | 2 | 3 | 5 | 6 | 10 |

### Conclusions

Based on the analysis of distributions and descriptive statistics with the cooperation of SI therapists, a normalization of test and questionnaire scales has been created.

The results of the Test of Motor Disorders with Sensory Basis dictate that a miscomprehension could have occurred in the way therapists mark answers for the scale for praxes and visual-motor coordination disorders. Data analyzed in this way may show false results. It is proposed to define the ranges of specific indicators of this test on an ad hoc basis or to adopt the present ranges and appreciate them when carrying out a subsequent analysis on a new database.

Indicators included in the questionnaire have been defined.

The given ranges of particular indicators may be more fully defined in terms of age groups, following statistics of particular indicators in these groups.

Results of the correlation analysis give a range of information about statistically significant relations between the indicators that also may be helpful when estimating the indicators. A deeper analysis is left for SI therapy specialists.

It should be emphasized that in the group of children without disorders, the degree of some indicators was close to extreme results. Therefore, a question arises what was the reason that they had not been classified into the groups of children with disorders? The possible answers may be the following:
- A therapist needed to confirm a disorder in several fields to diagnose the disorder.
- A therapist verified the test results by observing the child and the observation did not confirm the disorders.
- A therapist did not do the test earnestly.

Each time after approving particular scales of dividing the indicators into the norm, a risk and disorders, one should verify how the prevalence rates were distributed in the groups of persons with and without disorders.

## Claims

1. A system for generating a user's rehabilitation plan comprising a step of assigning the user to a diagnostic profile assigned to a defined measurement scale of sensory processing disorders, communicating with a computer network with the use of computer-based devices (402-408) and accessing to data resources (416, 418) by means of access means (414), comprising:
c) Obtaining (108) unit data related to a degree of sensory processing disorders, comprising user's unit data retrieved from:
i) a Test of Sensory Processing Disorders,
ii) a questionnaire,
d) Processing (110) the unit data in order to generate at least one set of computerized profile parameters of the degree of sensory processing disorder,
e) Obtaining (114) reference/control data including at least one set of predefined parameters corresponding to at least one defined measurement scale,
f) Comparing (116) at least one set of computerized profile parameters with at least one set of predefined parameters corresponding to at least one measurement scale in order to establish a degree of the user's sensory processing disorder,
g) Assigning (118) the diagnostic profile corresponding to at least one value on the measurement scale to at least one set of computerized user profile parameters,
h) Generating (120) a rehabilitation plan for the user assigned to the given diagnostic profile using a computer-based device (402-408).

2. The system according to claim 1, wherein the common access means (414) sends, to the computer-based device (402-408) associated with the user, an electronic invitation (100) to perform a user electronic questionnaire/reconnaissance via a network, wherein every question of the user electronic questionnaire/reconnaissance is configured to measure behavioral features of the user and to assign them to a defined measurement scale of sensory processing disorders.

3. The system according to claim 1, including at least one additional step:
a) Obtaining (102) variables describing behavioral features of the user retrieved from the reconnaissance,
b) Processing (104) the variables describing behavioral features in order to qualify the user for further diagnostic tests and/or a consultation with a sensory integration therapist,
i) Generating (122) a user profile, wherein the user profile includes at least one element from the following: personal data, a degree of sensory processing disorder, a diagnosis, a set of computerized user profile parameters, a set of predefined parameters generated for the given user, a value of the measurement scale to which the given user is assigned, a diagnostic profile.

4. The system according to claim 1, wherein the reference/control data that consists of at least one set of predefined parameters assigned to a given value of the measurement scale is predefined and delivered by the common access means (414), according to a method as defined in the claims.

5. The system according to any one of the preceding claims, wherein the user profile is created using predefined elements of user profiles, and a user ID identifier is assigned to the user profile, wherein the user profile jest created (122) and stored on a server, wherein the server is a part of the common access means (414).

6. The system according to claim 1, wherein the unit data includes data related to the degree of the sensory processing disorder, such as:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii)balance (B),
viii) praxes (P),
ix) reflex integration (RI),
x) visual-motor coordination (VMC),
xi) distribution of antigravity muscles tension (DAMT).

7. The system according to any one of the preceding claims, wherein the diagnostic profile corresponds to the user age category and/or is generated for a defined time period, wherein a rehabilitation plan (502) assigned to the generated diagnostic profile includes at least one rehabilitation exercise for at least one of the following sensory processing disorders:
i) tactile reactivity (TR),
ii) vestibular reactivity (VeR),
iii) auditory reactivity (AR),
iv) visual reactivity (ViR),
v) olfactory reactivity (OR),
vi) oculomotor control (OC),
vii) balance (B),
viii) praxes (P),
ix) reflex integration (RI),
x) visual-motor coordination (VMC),
xi) distribution of antigravity muscles tension (DAMT).

8. The system according to any one of the preceding claims, including at least one additional step of the following:
- The diagnostic profile, including the effects of performing the set of rehabilitation exercises along with the assigned thereto rehabilitation plan is reported (126) periodically to the common access means (414) in the form of a computer file containing the user ID identifier,
- After a predefined time period, a control test is generated (128) verifying the effects of performing the set of rehabilitation exercises of the given diagnostic profile assigned to the given user,
- Analyzing (132) the reported diagnostic profiles, including the effects of performing the set of rehabilitation exercises along with the assigned thereto rehabilitation plan and/or results of the users' control tests, wherein the rehabilitation progress of the users belonging to the same age category are grouped into several groups - groups assigned to the same or a similar value on the measurement scale and/or to a given diagnostic profile,
- Updating (134) the diagnostic profile based on assigning:
- the user to another value on the measurement scale, and/or
- the user to another diagnostic profile, and/or
- the rehabilitation exercises to a new value on the measurement scale of sensory processing disorders, and/or
- adding at least one new rehabilitation exercise to the given diagnostic profile,
Wherein the step of updating (134) the diagnostic profile is executed in databases of the common access means and is transferred to the user's computer-based device (402-408) by the common access means (414) in the form of a computer file containing the user ID identifier,
- generating (136) a new diagnostic profile and a new, assigned thereto, set of rehabilitation exercises and/or extending the execution time for this diagnostic profile.

9. The system according to any one of the preceding claims, wherein the correctness of the execution of the rehabilitation exercise is assessed according to a predefined point scale.

10. A method for assigning profile parameters of sensory processing disorders corresponding to at least one value on a measurement (diagnostic) scale of sensory processing disorders, accessing (208) data resources (416, 418) in the common access means (414) storing sets of predefined profile parameters of many users, comprising steps of:
a) receiving a user's unit data (520) and assigning a first weight thereto (200), wherein the user's unit data (520) is data retrieved from the Test of Sensory Processing Disorders and/or the user's personal questionnaire,
b) receiving data about the number of users and assigning a second weight thereto (202), wherein, when calculating (202) the number of users, only users are counted who have a similar degree sensory processing disorders or are assigned to the same or similar value on the measurement scale as the given user,
c) receiving data about the age of the users and assigning a third weight thereto (204), wherein when calculating (204) the user's age, only users are counted who have a similar degree of sensory processing disorders or are assigned to the same value on the measurement scale as the given user and/or assigned to the same or similar age category,
d) receiving data about the duration of the user's therapy and assigning a fourth weight thereto (206),
e) calculating (210) reference/control data being at least one set of predefined profile parameters for the given user corresponding to at least one value on a defined measurement scale based on the user unit data, the number of users, the age of users, the therapy duration, and the assigned weights,
f) generating (214) reference/control data for the given user, and/or
g) receiving data about the sets of rehabilitation exercises established for the given diagnostic profile corresponding to a selected value on a defined measurement scale and assigning a fifth weight thereto (212).

11. The method according to any one of the preceding claims, wherein
- At least one set of computerized profile parameters of the degree of the user's sensory processing disorders is generated (216) from the user's unit data (520), wherein the at least one computerized profile parameter of the degree of the sensory processing disorder from the test is generated (216) separately for: tactile reactivity (TR), vestibular reactivity (VeR), auditory reactivity (AR), visual reactivity (ViR), olfactory reactivity (OR), oculomotor control (OC), balance (B), praxes (P), reflex integration (RI), visual-motor coordination (VMC), and distribution of antigravity muscles tension (DAMT);
- If the computerized profile parameters of the degree of the sensory processing disorder of a given user can't be assigned (218) to any value on the measurement scale, a new value is created (220) on the measurement scale, and to this new value, computerized profile parameters of the degree of the user's sensory processing disorders are assigned (222) that are then preliminarily defined (224) and reported (226) to data resources (416, 418) of the common access means (414).

12. The method according to claim 10, wherein the computerized profile parameters of the degree of the user sensory processing disorders are defined based on expert knowledge.

13. The method according to any one of the preceding claims, wherein the computerized profile parameters of the degree of the user sensory processing disorders retrieved from the unit data (520) of a new user assigned to a value on the measurement scale are included when calculating (210) at least one set of predefined parameters corresponding to at least one value on the defined measurement scale for a subsequent user.

14. The method according to any one of the preceding claims, wherein the age of the users is divided into four age categories: 12-23 months; 24-35 months; 36-47 months; and 48-59 months.

15. The method according to any one of the preceding claims, wherein also information is generated about the number of users who are of the same age, who have the same value assigned on the measurement scale, and who have the same diagnostic profile assigned as the given user, wherein the information about the number of users, their ages, their assignment to the same value on the measurement scale, the diagnostic profile, therapy duration, and the therapy effects are stored in the common access means (414).
